(19) Europäisches Patentamt
European Patent Office
Office européen des brevets

(11) Veröffentlichungsnummer: **0 529 451 A1**

# (12) EUROPÄISCHE PATENTANMELDUNG

(21) Anmeldenummer: **92113957.2**

(22) Anmeldetag: **17.08.92**

(51) Int. Cl.[5]: **C07D 231/06**, A01N 47/38, C07D 403/04

(30) Priorität: **28.08.91 DE 4128564**
**29.05.92 DE 4217863**

(43) Veröffentlichungstag der Anmeldung:
**03.03.93 Patentblatt 93/09**

(84) Benannte Vertragsstaaten:
**BE CH DE ES FR GB IT LI NL**

(71) Anmelder: **BAYER AG**

**W-5090 Leverkusen 1 Bayerwerk(DE)**

(72) Erfinder: **Kanellakopulos, Johannes, Dr.**
**Mozartstrasse 94c**
**W-4010 Hilden(DE)**
Erfinder: **Fuchs, Rainer, Dr.**
**Am Rohm 107**
**W-5600 Wuppertal 1(DE)**
Erfinder: **Erdelen, Christoph, Dr.**
**Unterbüscherhof 22**
**W-5653 Leichlingen 1(DE)**

## (54) Substituierte Pyrazoline als Schädlingsbekämpfungsmittel.

(57) Beschrieben werden neue Pyrazoline der Formel (I)

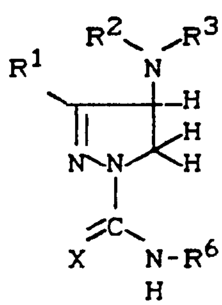

(I)

in welcher X, $R^1$, $R^2$, $R^3$ und $R^6$ die in der Beschreibung angegebene Bedeutung haben, sowie ein Verfahren zu deren Herstellung.

Die neuen Pyrazoline werden als Schädlingsbekämpfungsmittel verwendet.

EP 0 529 451 A1

Die Erfindung betrifft neue, substituierte Pyrazoline, mehrere Verfahren zu ihrer Herstellung und ihre Verwendung als Schädlingsbekämpfungsmittel.

Es ist bekannt, daß bestimmte substituierte Pyrazoline insektizide Eigenschaften besitzen (vgl. z.B. DE-A 2700258; DE-A 2529689; US-A 4.174.393).

Die Wirkungshöhe bzw. Wirkungsdauer dieser vorbekannten Verbindungen ist jedoch, insbesondere bei bestimmten Insekten oder bei niedrigen Anwendungskonzentrationen nicht in allen Anwendungsgebieten völlig zufriedenstellend.

Es wurden neue substituierte Pyrazoline der allgemeinen Formel (I) gefunden

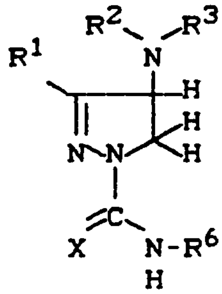

(I)

in welcher X, $R^1$, $R^2$, $R^3$ und $R^6$ jeweils folgende Bedeutungskombinationen einnehmen können:

| $R^1$ | $R^2$ | $R^3$ | $R^6$ | X |
|---|---|---|---|---|
| 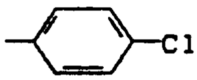 | 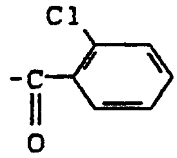 | H | 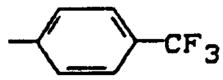 | O |
| 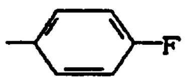 | 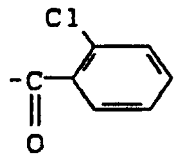 | H | 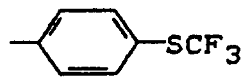 | O |
| 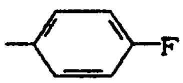 | 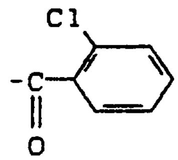 | H | 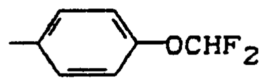 | O |
| 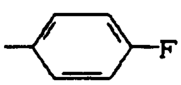 | 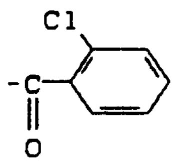 | H | 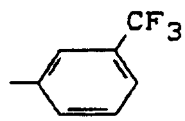 | O |
| 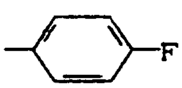 | 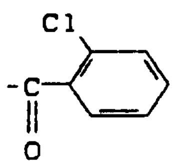 | H | 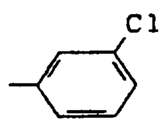 | O |
| 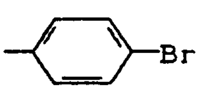 | 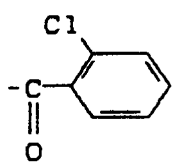 | H | 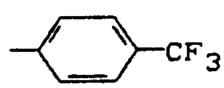 | O |
| 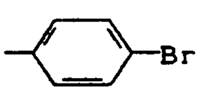 | 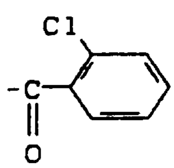 | H | 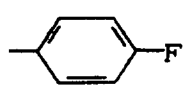 | O |

| $R^1$ | $R^2$ | $R^3$ | $R^6$ | X |
|---|---|---|---|---|
| 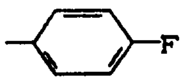 | 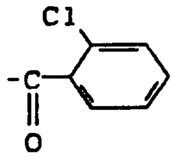 | H | 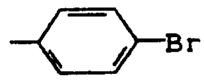 | O |
| 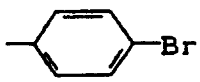 | 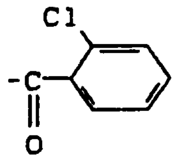 | H | 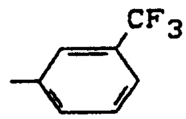 | O |
| 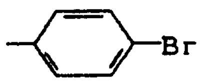 | 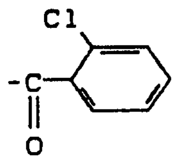 | H | 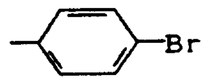 | O |
| 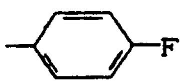 | 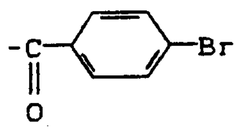 | H | 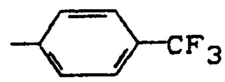 | O |
| 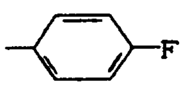 | 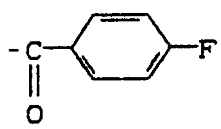 | H | 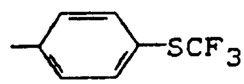 | O |
| 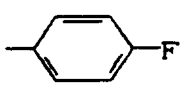 | 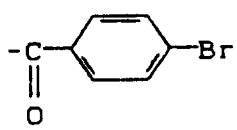 | H | 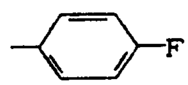 | O |
| 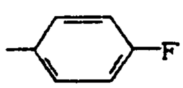 | 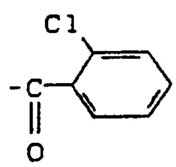 | H | 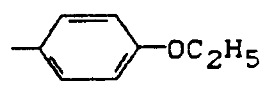 | O |
| 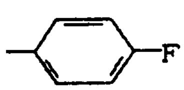 | 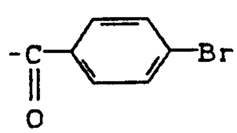 | H | 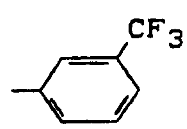 | O |

| $R^1$ | $R^2$ | $R^3$ | $R^6$ | X |
|---|---|---|---|---|
| 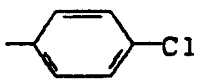 | 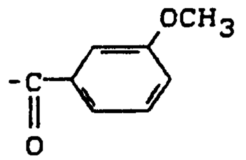 | H | 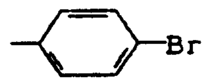 | O |
| 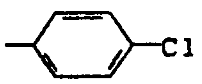 | 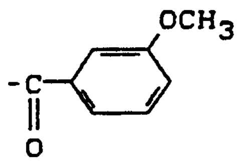 | H | 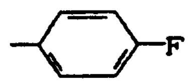 | O |
| 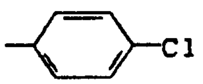 | 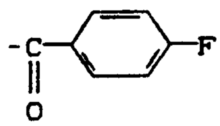 | H | 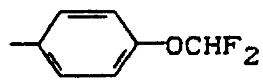 | O |
| 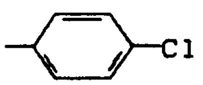 | 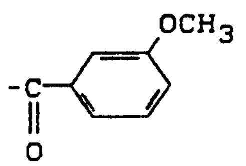 | H | 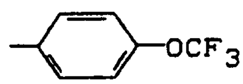 | O |
| 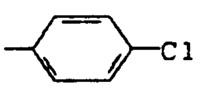 | 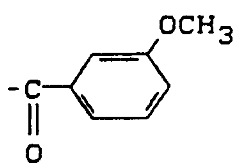 | H | 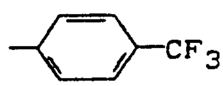 | O |
| 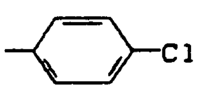 | 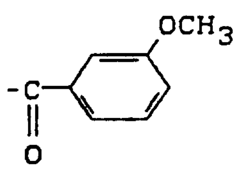 | H | 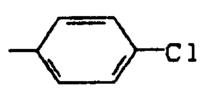 | O |
| 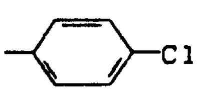 | 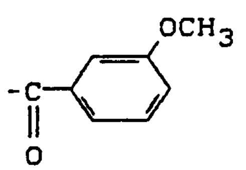 | H | 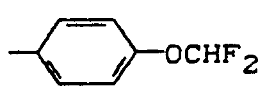 | O |
| 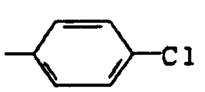 | 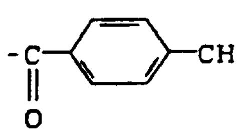 | H | 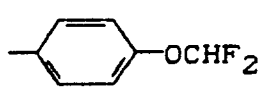 | O |

| $R^1$ | $R^2$ | $R^3$ | $R^6$ | X |
|---|---|---|---|---|
| 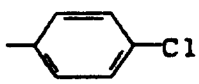 | 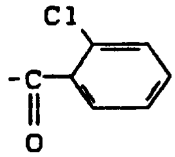 | H | 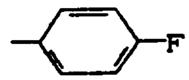 | O |
| 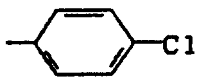 | 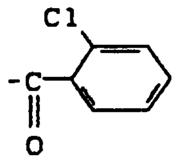 | H | 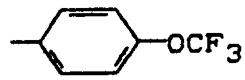 | O |
| 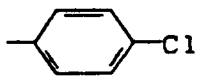 | 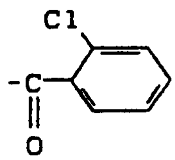 | H | 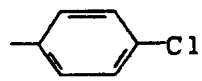 | O |
| 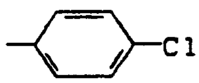 | 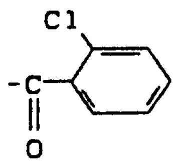 | H | 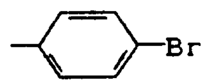 | O |
| 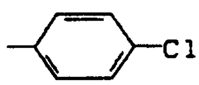 | 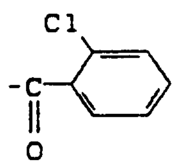 | H | 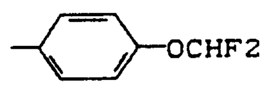 | O |
| 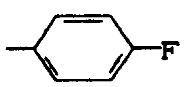 | 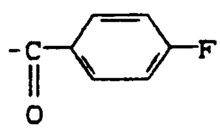 | H | 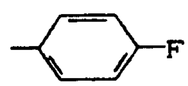 | O |
| 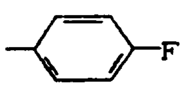 | 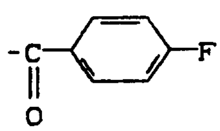 | H | 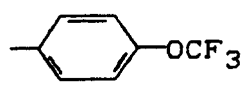 | O |
| 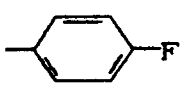 | 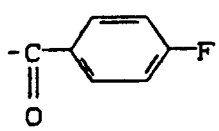 | H | 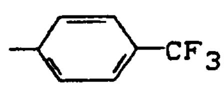 | O |

| $R^1$ | $R^2$ | $R^3$ | $R^6$ | X |
|---|---|---|---|---|
| 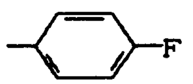 | 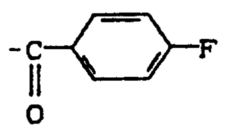 | H | 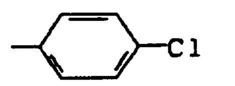 | O |
| 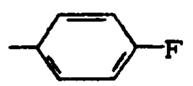 | 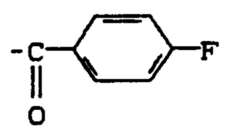 | H | 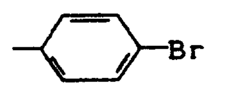 | O |
| 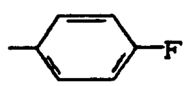 | 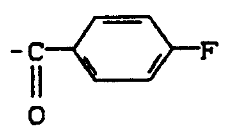 | H | 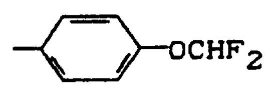 | O |
| 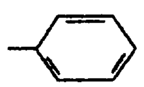 | 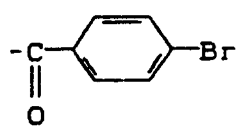 | H | 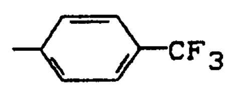 | O |
| 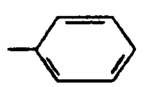 | 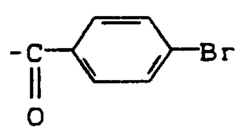 | H | 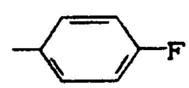 | O |
| 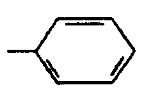 | 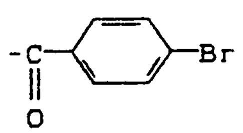 | H | 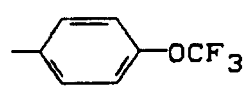 | O |
| 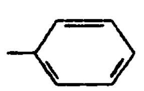 | 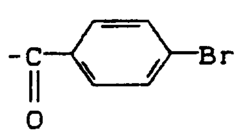 | H | 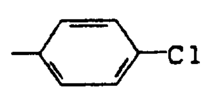 | O |
| 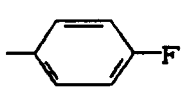 | 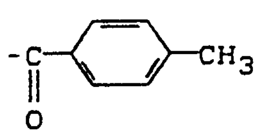 | H | 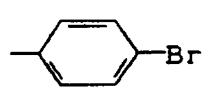 | O |
| 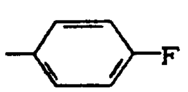 | 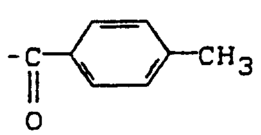 | H | 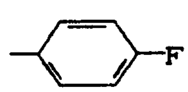 | O |

| $R^1$ | $R^2$ | $R^3$ | $R^6$ | X |
|---|---|---|---|---|
| 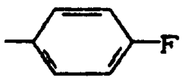 | 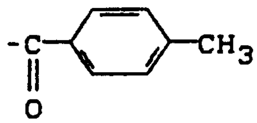 | H | 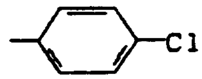 | O |
| 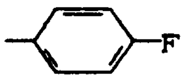 | 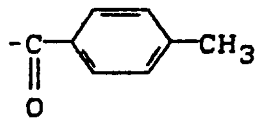 | H | 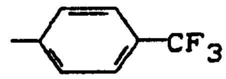 | O |
| 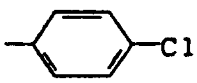 | 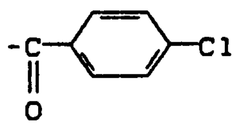 | H | 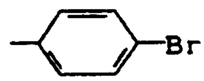 | O |
| 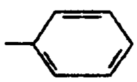 | 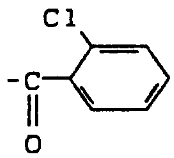 | H | 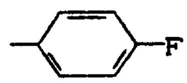 | O |
| 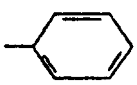 | 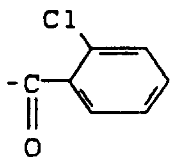 | H | 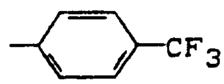 | O |
| 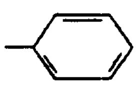 | 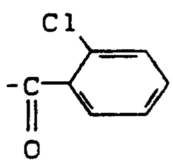 | H | 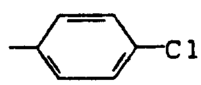 | O |
| 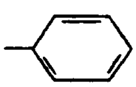 | 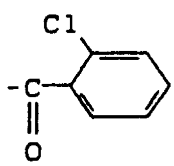 | H | 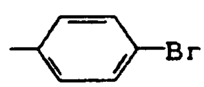 | O |
| 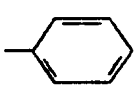 | 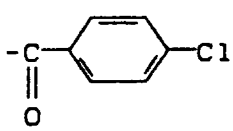 | H | 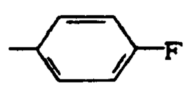 | O |

| $R^1$ | $R^2$ | $R^3$ | $R^6$ | X |
|---|---|---|---|---|
| 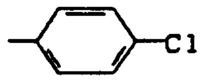 | 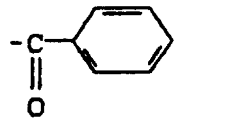 | H | 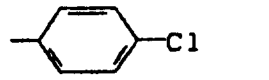 | O |
| 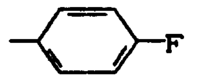 | 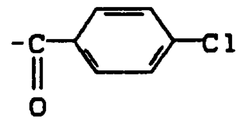 | H | 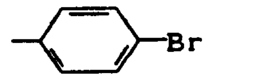 | O |
| 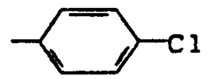 | 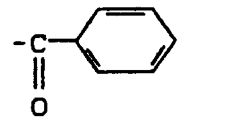 | H | 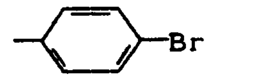 | O |
| 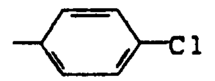 | 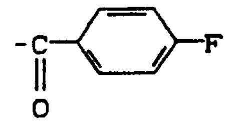 | H | 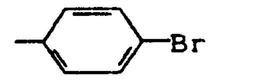 | O |
| 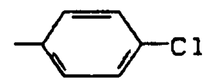 | 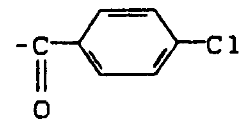 | H | 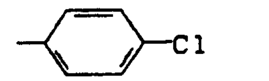 | O |
| 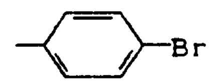 | 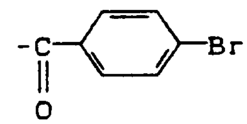 | H | 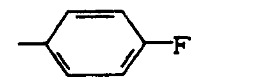 | O |
| 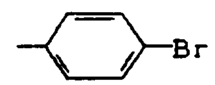 | 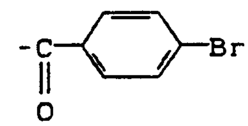 | H | 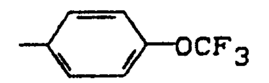 | O |
| 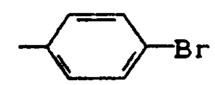 | 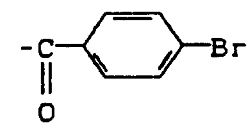 | H | 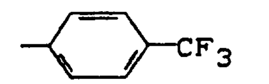 | O |
| 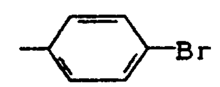 | 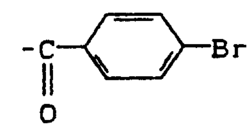 | H | 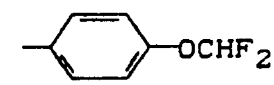 | O |

| $R^1$ | $R^2$ | $R^3$ | $R^6$ | X |
|---|---|---|---|---|
| 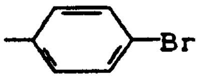 | 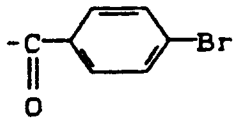 | H | 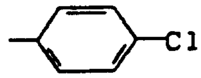 | O |
| 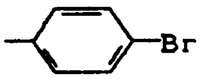 | 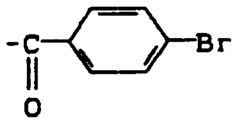 | H | 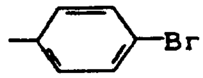 | O |
| 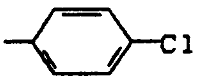 | 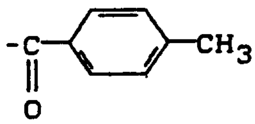 | H | 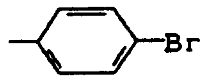 | O |
| 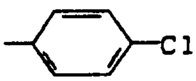 | 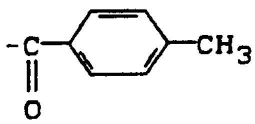 | H | 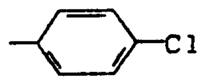 | O |
| 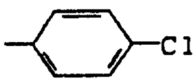 | 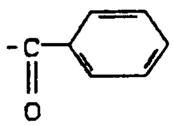 | H | 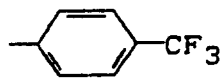 | O |
| 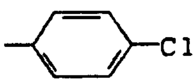 | 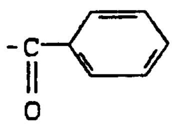 | H | 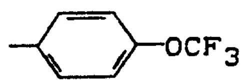 | O |
| 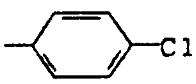 | 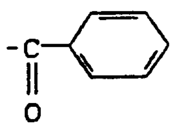 | H | 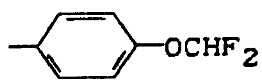 | O |
| 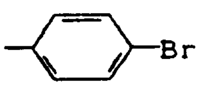 | 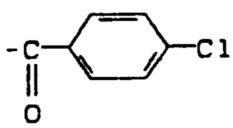 | H | 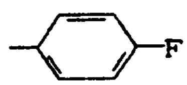 | O |
| 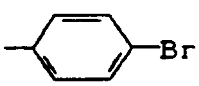 | 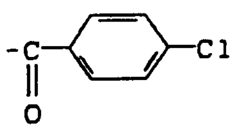 | H | 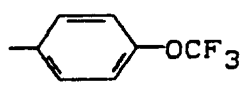 | O |

| $R^1$ | $R^2$ | $R^3$ | $R^6$ | x |
|---|---|---|---|---|
| 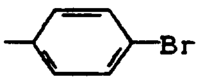 | 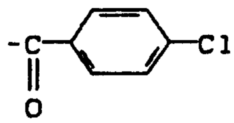 | H | 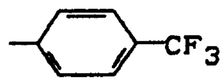 | 0 |
| 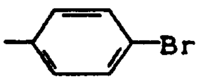 | 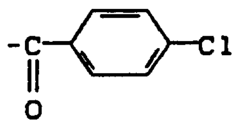 | H | 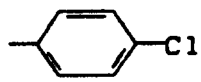 | 0 |
| 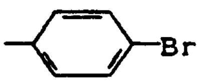 | 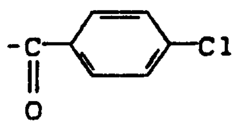 | H | 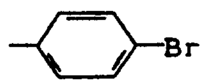 | 0 |
| 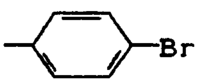 | 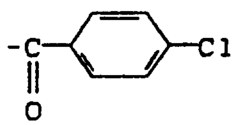 | H | 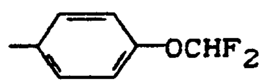 | 0 |
| 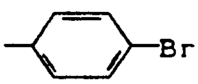 | 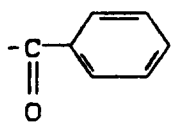 | H | 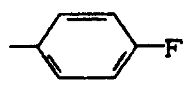 | 0 |
| 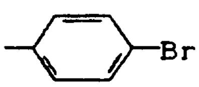 | 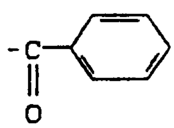 | H | 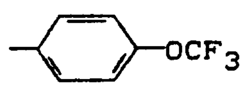 | 0 |
| 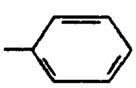 | 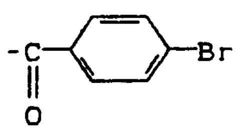 | H | 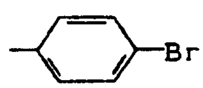 | 0 |
| 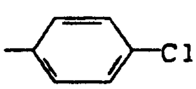 | 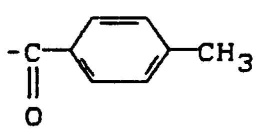 | H | 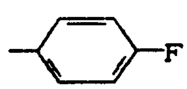 | 0 |
| 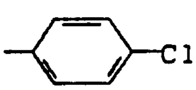 | 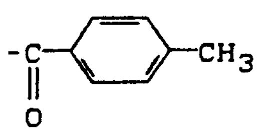 | H | 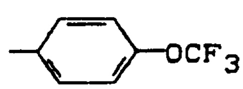 | 0 |

| $R^1$ | $R^2$ | $R^3$ | $R^6$ | X |
|---|---|---|---|---|
| 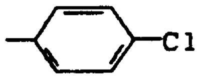 | 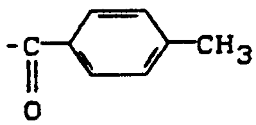 | H | 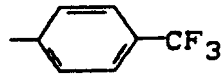 | O |
| 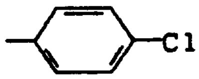 | 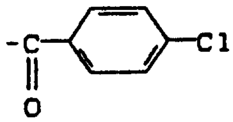 | H | 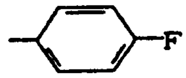 | O |
| 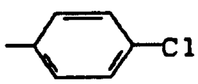 | 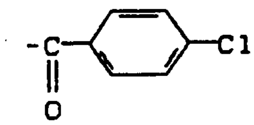 | H | 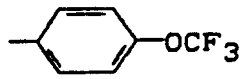 | O |
| 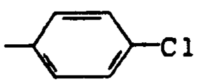 | 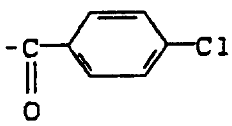 | H | 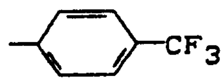 | O |
| 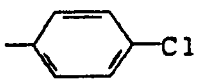 | 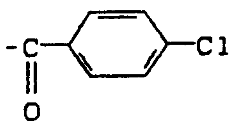 | H | 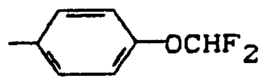 | O |
| 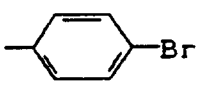 | 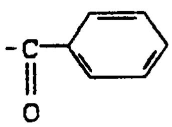 | H | 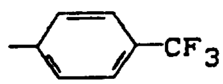 | O |
| 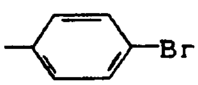 | 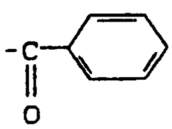 | H | 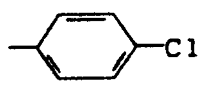 | O |
| 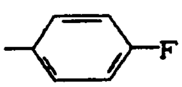 | 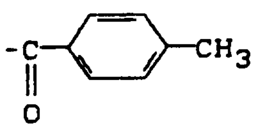 | H | 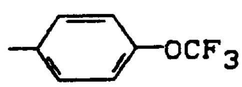 | O |
| 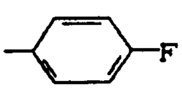 | 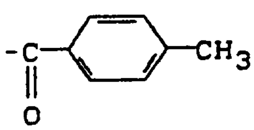 | H | 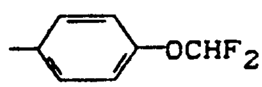 | O |

| $R^1$ | $R^2$ | $R^3$ | $R^6$ | X |
|---|---|---|---|---|
| 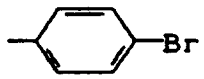 | 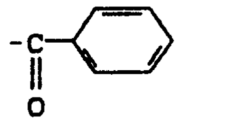 | H | 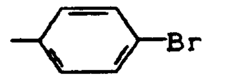 | O |
| 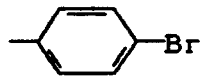 | 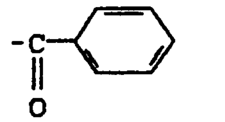 | H | 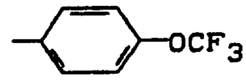 | O |
| 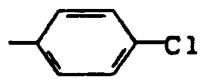 | 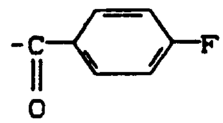 | H | 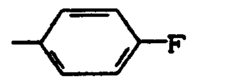 | O |
| 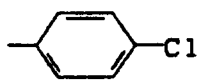 | 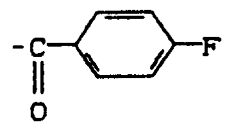 | H | 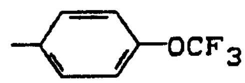 | O |
| 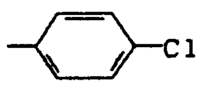 | 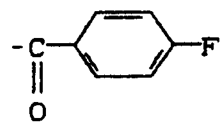 | H | 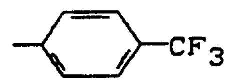 | O |
| 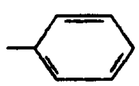 | 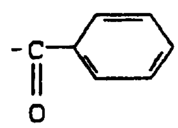 | H | 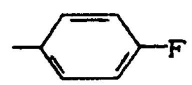 | O |
| 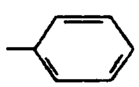 | 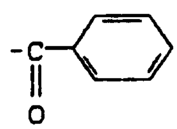 | H | 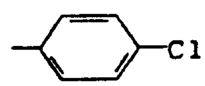 | O |
| 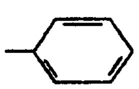 | 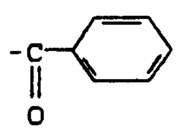 | H | 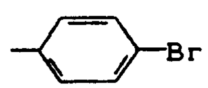 | O |
| 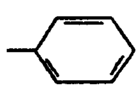 | 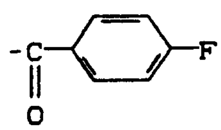 | H | 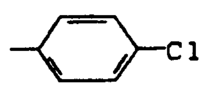 | O |

| $R^1$ | $R^2$ | $R^3$ | $R^6$ | X |
|---|---|---|---|---|
| 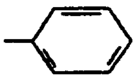 | 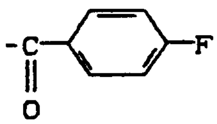 | H | 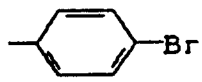 | O |
| 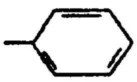 | 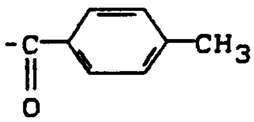 | H | 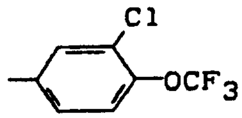 | O |
| 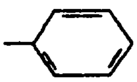 | 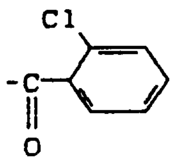 | H | 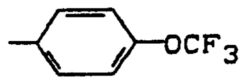 | O |
| 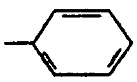 | 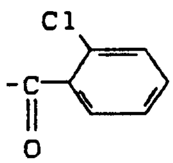 | H | 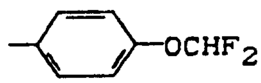 | O |
| 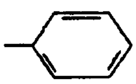 | 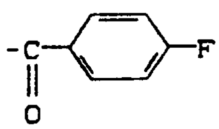 | H | 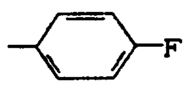 | O |
| 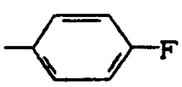 | 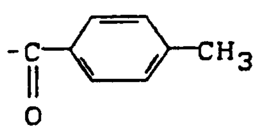 | H | 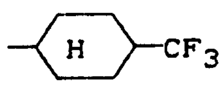 | O |
| 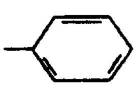 | 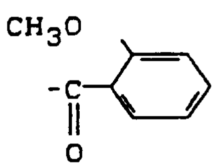 | H | 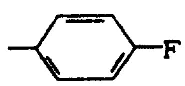 | O |
| 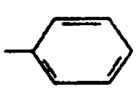 | 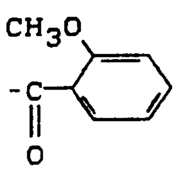 | H | 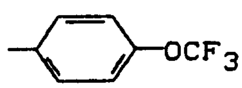 | O |

| $R^1$ | $R^2$ | $R^3$ | $R^6$ | X |
|---|---|---|---|---|
| 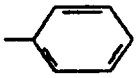 | 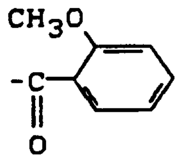 | H | 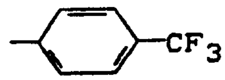 | O |
| 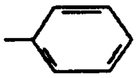 | 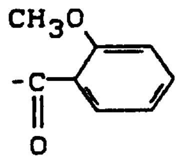 | H | 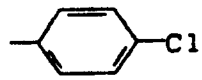 | O |
| 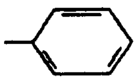 | 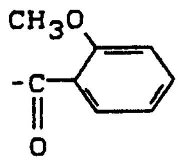 | H | 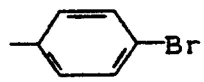 | O |
| 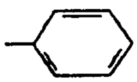 | 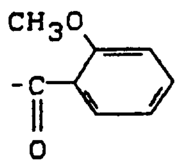 | H | 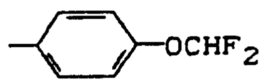 | O |
| 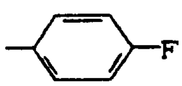 | 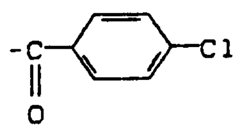 | H | 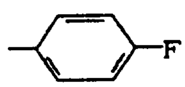 | O |
| 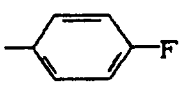 | 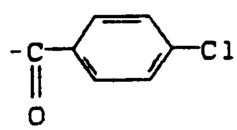 | H | 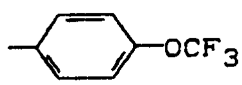 | O |
| 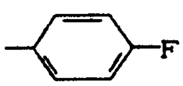 | 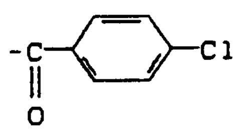 | H | 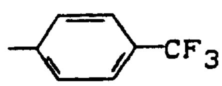 | O |
| 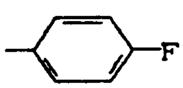 | 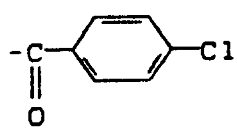 | H | 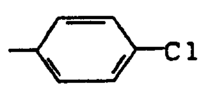 | O |

| $R^1$ | $R^2$ | $R^3$ | $R^6$ | X |
|---|---|---|---|---|
| 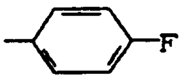 | 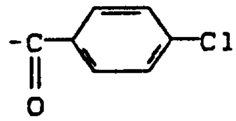 | H | 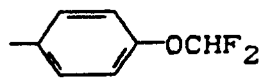 | O |
| 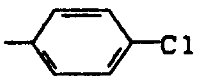 | 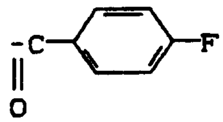 | H | 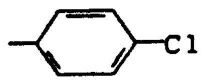 | O |
| 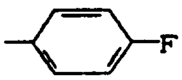 | 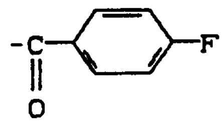 | H | 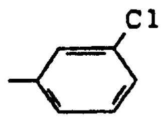 | O |
| 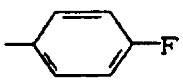 | 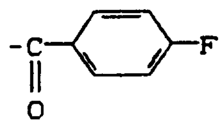 | H | 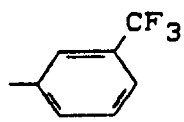 | O |
| 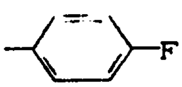 | 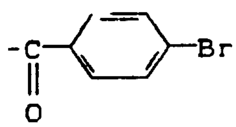 | H | 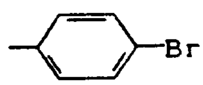 | O |
| 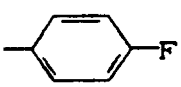 | 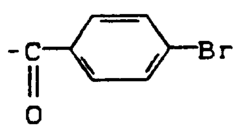 | H | 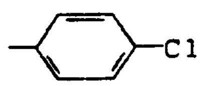 | O |
| 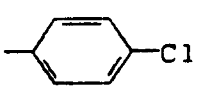 | 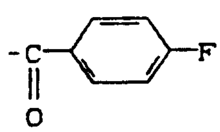 | H | 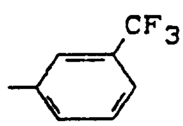 | O |
| 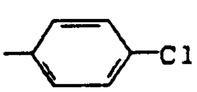 | 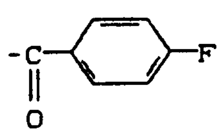 | H | 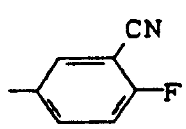 | O |

| $R^1$ | $R^2$ | $R^3$ | $R^6$ | X |
|---|---|---|---|---|
| 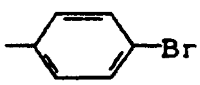 | 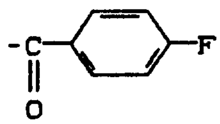 | H | 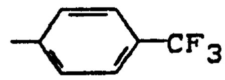 | O |
| 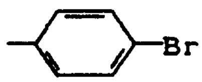 | 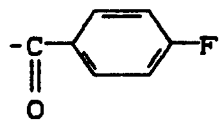 | H | 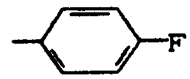 | O |
| 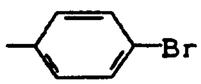 | 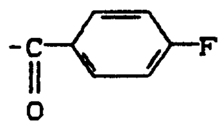 | H | 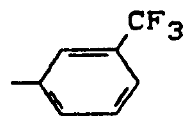 | O |
| 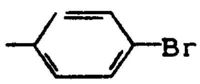 | 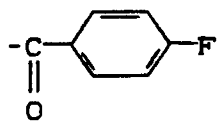 | H | 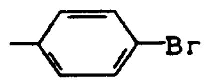 | O |
| 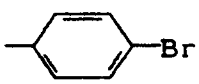 | 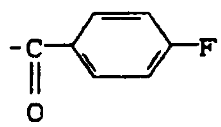 | H | 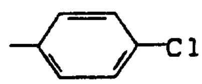 | O |
| 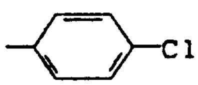 | 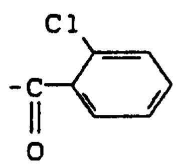 | H | 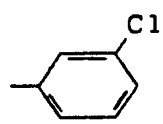 | O |
| 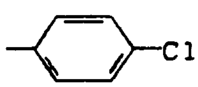 | 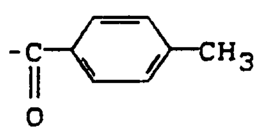 | H | 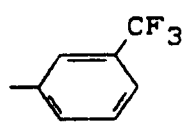 | O |
| 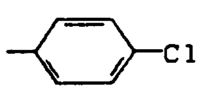 | 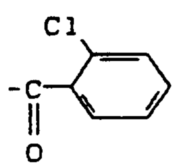 | H | 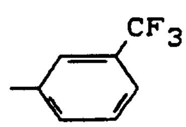 | O |

| $R^1$ | $R^2$ | $R^3$ | $R^6$ | X |
|---|---|---|---|---|
| 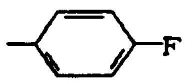 | 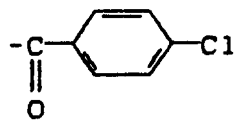 | H | 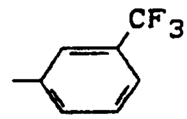 | O |
| 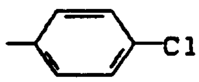 | 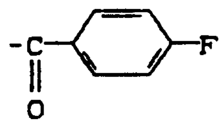 | H | 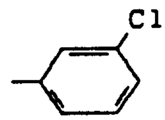 | O |
| 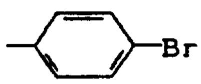 | | 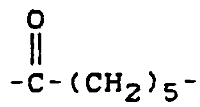 | 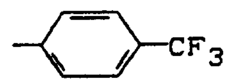 | O |
| 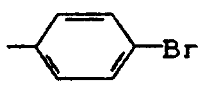 | 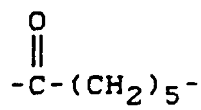 | | 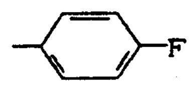 | O |
| 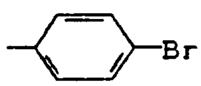 | 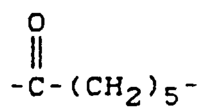 | | 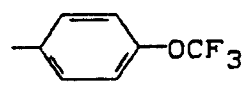 | O |
| 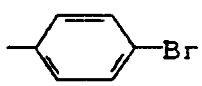 | 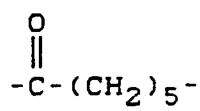 | | 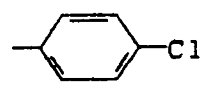 | O |
| 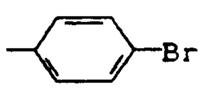 | 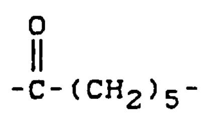 | | 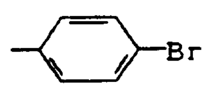 | O |
| 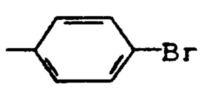 | 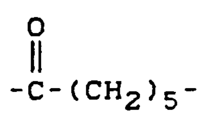 | | 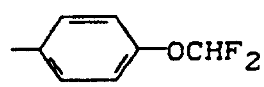 | O |

| $R^1$ | $R^2$ | $R^3$ | $R^6$ | X |
|---|---|---|---|---|
| 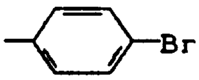 | 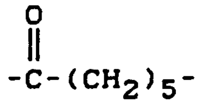 | | 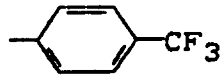 | O |
| 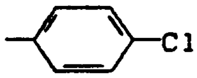 | 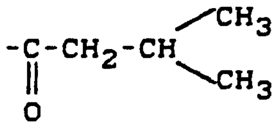 | H | 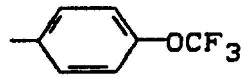 | O |
| 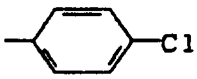 | 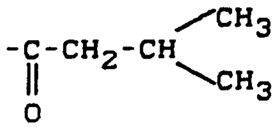 | H | 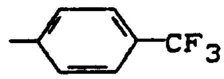 | O |
| 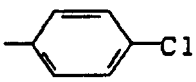 | 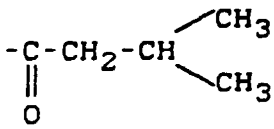 | H | 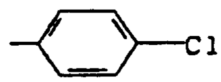 | O |
| 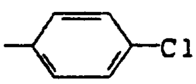 | 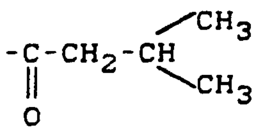 | H | 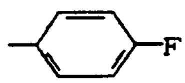 | O |
| 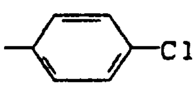 | 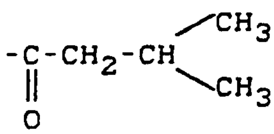 | H | 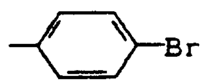 | O |
| 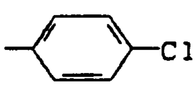 | 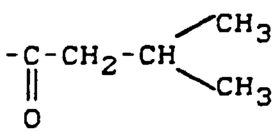 | H | 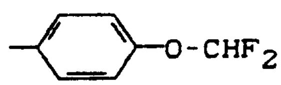 | O |
| 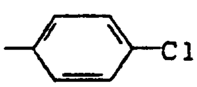 | 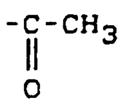 | H | 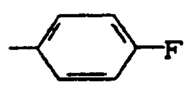 | O |
| 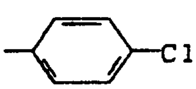 | 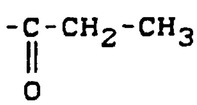 | H | 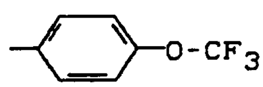 | O |

| $R^1$ | $R^2$ | $R^3$ | $R^6$ | X |
|---|---|---|---|---|
| 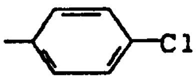 | 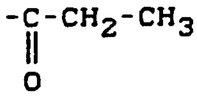 | H | 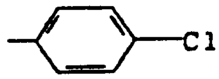 | O |
| 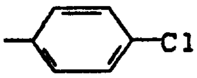 | 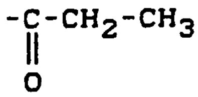 | H | 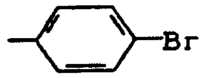 | O |
| 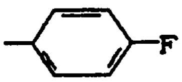 | 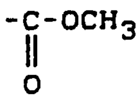 | H | 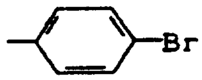 | O |
| 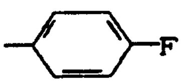 | 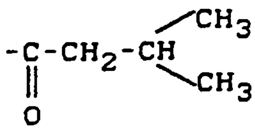 | H | 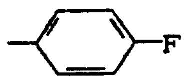 | O |
| 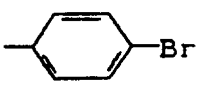 | 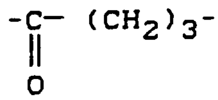 | | 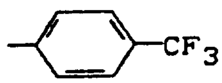 | O |
| 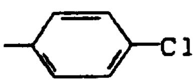 | 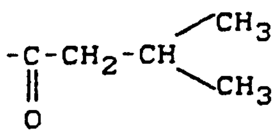 | H | 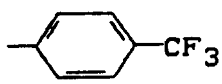 | O |
| 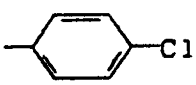 | 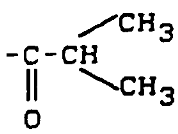 | H | 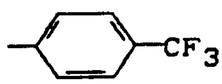 | O |
| 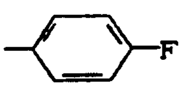 | 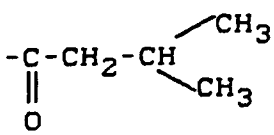 | H | 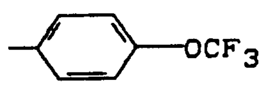 | O |
| 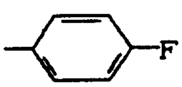 | 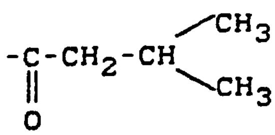 | H | 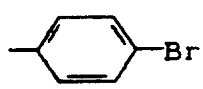 | O |

| $R^1$ | $R^2$ | $R^3$ | $R^6$ | X |
|---|---|---|---|---|
| 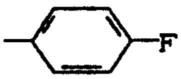 | 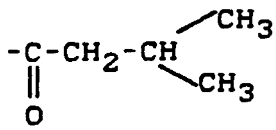 | H | 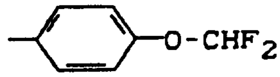 | O |
| 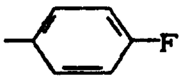 | 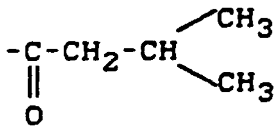 | H | 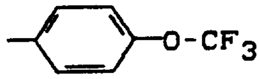 | O |
| 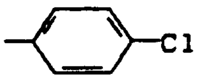 | 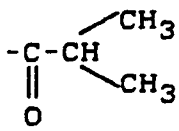 | H | 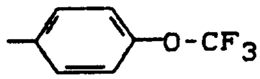 | O |
| 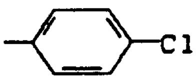 | 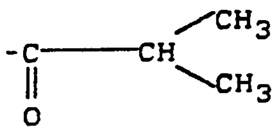 | H | 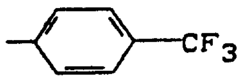 | O |
| 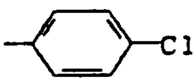 | 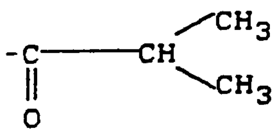 | H | 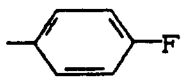 | O |
| 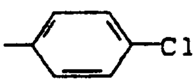 | 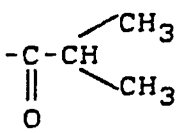 | H | 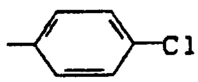 | O |
| 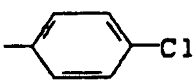 | 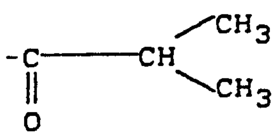 | H | 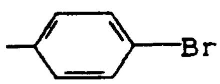 | O |
| 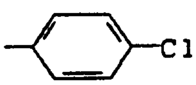 | 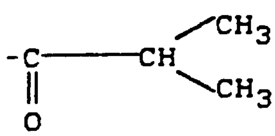 | H | 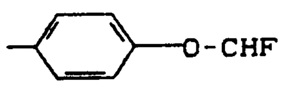 | O |
| 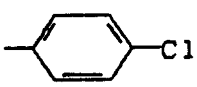 | 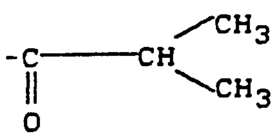 | H | 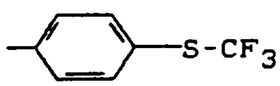 | O |

| $R^1$ | $R^2$ | $R^3$ | $R^6$ | X |
|---|---|---|---|---|
| 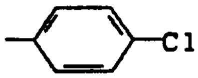 | 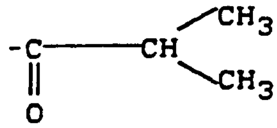 | H | 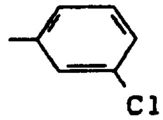 | O |
| 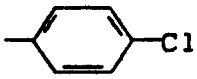 | 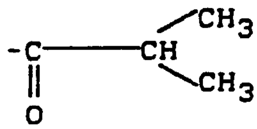 | H | 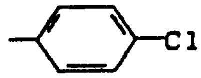 | O |
| 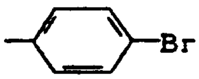 | 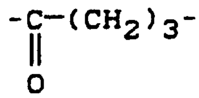 | | 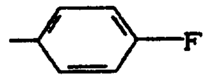 | O |
| 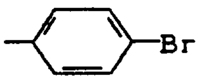 | 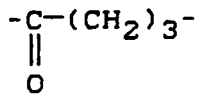 | | 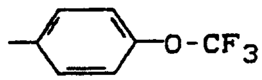 | O |
| 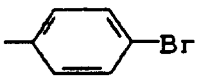 | 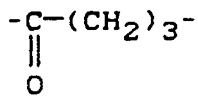 | | 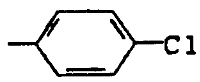 | O |
| 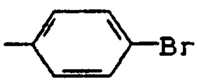 | 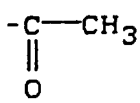 | H | 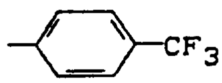 | O |
| 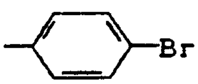 | 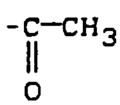 | H | 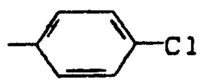 | O |
| 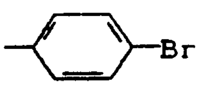 | 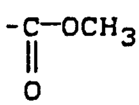 | H | 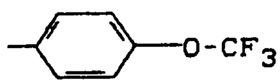 | O |
| 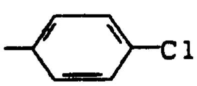 | 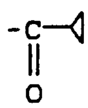 | H | 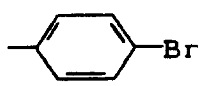 | O |

| $R^1$ | $R^2$ | $R^3$ | $R^6$ | X |
|---|---|---|---|---|
| 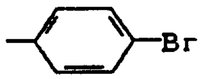 | | 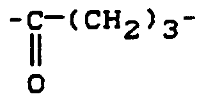 | 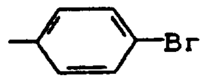 | O |
| 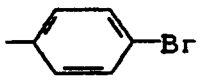 | | 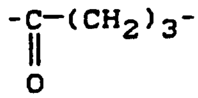 | 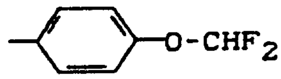 | O |
| 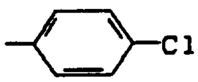 | 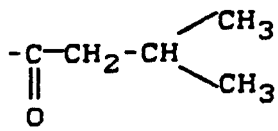 | H | 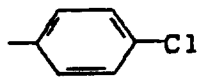 | O |
| 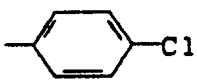 | 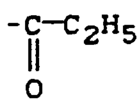 | H | 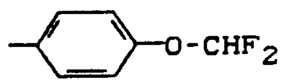 | O |
| 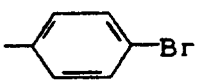 | 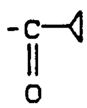 | H | 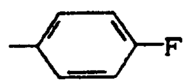 | O |
| 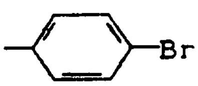 | 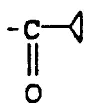 | H | 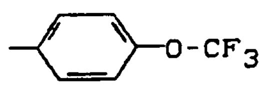 | O |
| 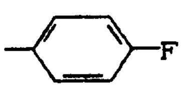 | 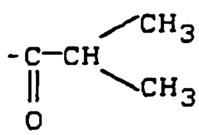 | H | 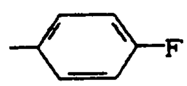 | O |
| 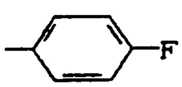 | 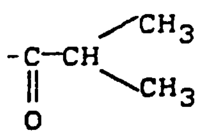 | H | 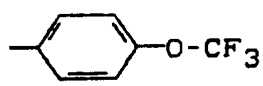 | O |
| 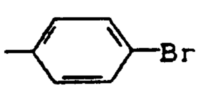 | 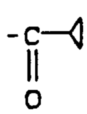 | H | 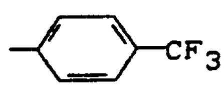 | O |

| $R^1$ | $R^2$ | $R^3$ | $R^6$ | X |
|---|---|---|---|---|
| 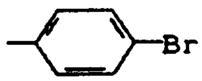 | 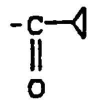 | H | 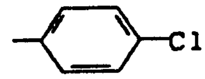 | O |
| 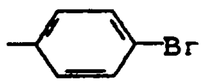 | 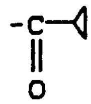 | H | 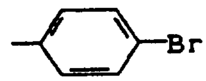 | O |
| 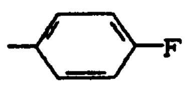 | 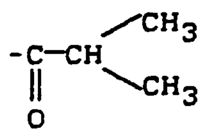 | H | 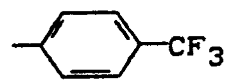 | O |
| 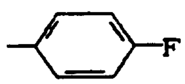 | 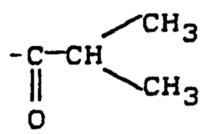 | H | 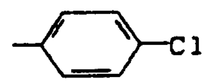 | O |
| 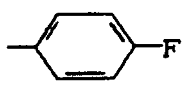 | 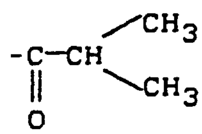 | H | 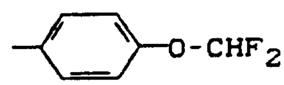 | O |
| 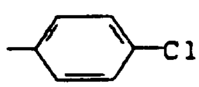 | 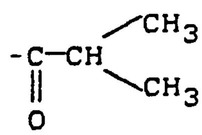 | H | 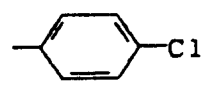 | S |
| 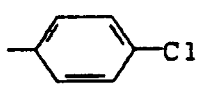 | 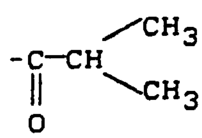 | H | 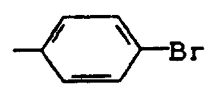 | S |

Weiterhin wurde gefunden, daß man die neuen substituierten Pyrazoline der allgemeinen Formel (I)

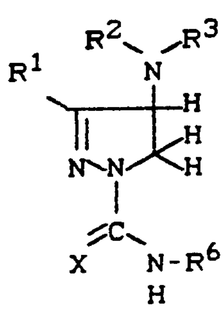

(I)

in welcher die Substituenten X, $R^1$, $R^2$, $R^3$ und $R^6$ die oben angegebene Bedeutung besitzen, erhält, wenn man in 1-Stellung unsubstituierte Pyrazoline der Formel (II),

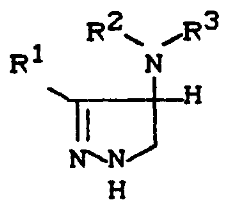

(II)

in welcher
$R^1$, $R^2$ und $R^3$ die oben angegebene Bedeutung haben,
entweder
a) mit Isocyanaten der Formel (III),

$R^6$-N=C=X (III)

in welcher
$R^6$ die oben angegebene Bedeutung haben,
oder
b) mit Carbaminsäurechloriden der Formel (IV),

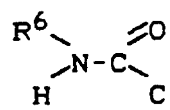

(IV)

in welcher
$R^6$ die oben angegebene Bedeutung hat,
gegebenenfalls in Gegenwart eines Verdünnungsmittels und gegebenenfalls in Gegenwart eines Reaktionshilfsmittels umsetzt.

Schließlich wurde gefunden, daß die neuen substituierten Pyrazoline der allgemeinen Formel (I) gute Wirksamkeit gegen tierische Schädlinge besitzen.

Überraschenderweise zeigen die erfindungsgemäßen substituierten Pyrazoline der allgemeinen Formel (I) eine erheblich bessere insektizide Wirksamkeit im Vergleich zu den aus dem Stand der Technik bekannten chemisch und/oder wirkungsmäßig naheliegenden Verbindungen.

Im einzelnen sei auf die bei den Herstellungsbeispielen genannten Verbindungen verwiesen.

Verwendet man beispielsweise 3-(4-Chlorphenyl)-4-[(2-chlorphenyl)-carbonylamino]-4,5-dihydropyrazol und 4-Trifluormethyl-phenylisocyanat als Ausgangsstoff, so läßt sich der Reaktionsablauf des erfindungsgemäßen Verfahrens (a) durch das folgende Formelschema darstellen:

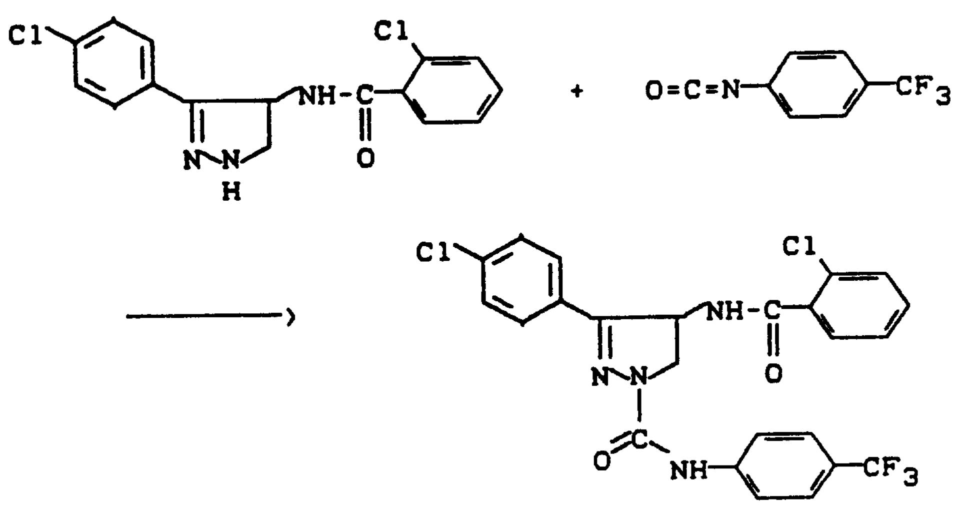

Verwendet man beispielsweise 3-(4-Chlorphenyl)-4-[(2-chlorphenyl)-carbonylamino]-4,5-dihydropyrazol und N-Methyl-N-(4-trifluormethylphenyl)-carbaminsäurechlorid als Ausgangsstoffe, so läßt sich der Reaktionsablauf des erfindungsgemäßen Verfahren (b) durch das folgende Formelschema darstellen:

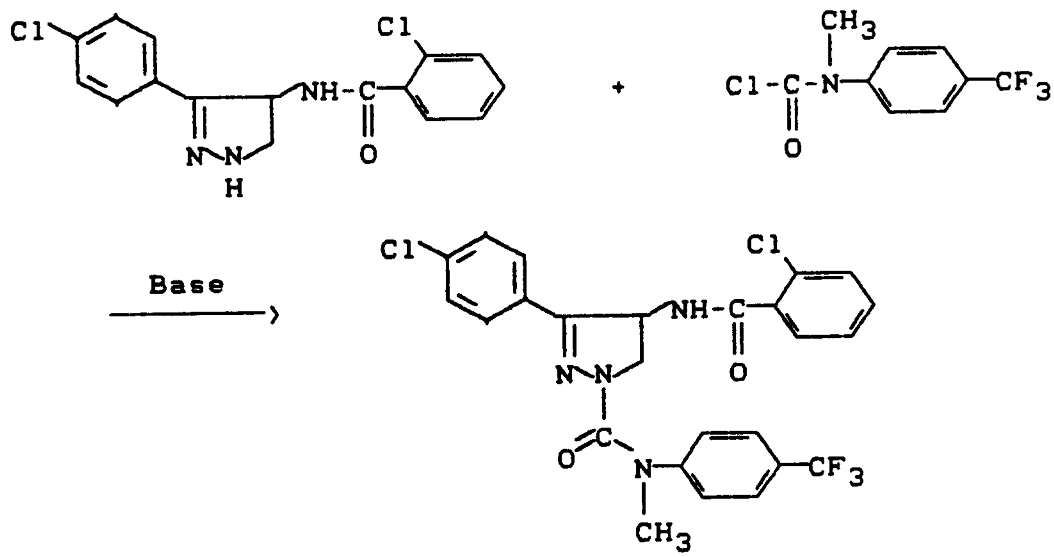


Die zur Durchführung des erfindungsgemäßen Verfahren (a) und (b) als Ausgangsstoffe benötigten in 1-Stellung unsubstituierten Pyrazoline sind durch die Formel (II) allgemein definiert. In dieser Formel (II) stehen $R^1$, $R^2$ und $R^3$ für diejenigen Reste, die bereits im Zusammenhang mit der Beschreibung der erfindungsgemäßen Verbindungen der Formel (I) genannt wurden.

Die in 1-Stellung unsubstituierten Pyrazoline der Formel (II) sind noch nicht bekannt. Man erhält sie beispielsweise, wenn man Phenacylaminderivate der Formel (V),

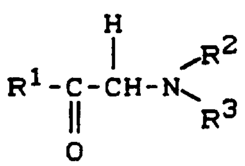

(V)

in welcher

$R^1$, $R^2$ und $R^3$ die oben angegebene Bedeutung haben,

mit Dimethylmethylenimmoniumchlorid der Formel (VI)

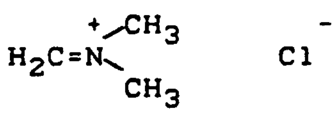

(VI)

gegebenenfalls in Gegenwart eines Verdünnungsmittels wie beispielsweise Acetonitril bei Temperaturen zwischen 30 ° C und 80 ° C umsetzt und anschließend in einer 2. Stufe die so erhältlichen Phenacyldiaminderivate der Formel (VII),

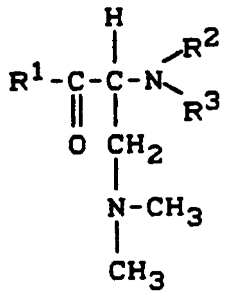 x HCl (VII)

in welcher

$R^1$, $R^2$ und $R^3$ die oben angegebene Bedeutung haben,

mit Hydrazinhydrat gegebenenfalls in Gegenwart eines Verdünnungsmittels wie beispielsweise Acetonitril bei Temperaturen zwischen 30 ° C und 80 ° C umsetzt.

Dabei ist es auch möglich und gegebenenfalls von Vorteil, die Umsetzung der Phenacylaminderivate der Formel (V) mit Dimethylmethylenimmoniumchlorid der Formel (VI) und die anschließende Umsetzung der so erhältlichen Phenacyldiaminderivate der Formel (VII) mit Hydrazinhydrat in einem Reaktionsschritt in einem sogenannten "Eintopfverfahren" durchzuführen.

Die Pyrazoline der Formel (II) erhält man alternativ auch, wenn man Phenacylaminderivate der Formel (Va),

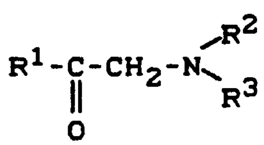 (Va)

in welcher

$R^1$, $R^2$ und $R^3$ die oben angegebene Bedeutung haben,

zunächst in einer ersten Stufe mit Formaldehyd gegebenenfalls in Gegenwart eines Verdünnungsmittels wie beispielsweise Ethanol bei Temperaturen zwischen 30 ° C und 80 ° C umsetzt und anschließend die so erhältlichen Phenacyl-Enamin-Derivate der Formel (VIII),

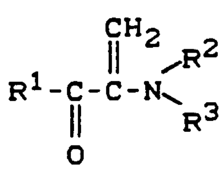 (VIII)

in welcher

$R^1$, $R^2$ und $R^3$ die oben genannte Bedeutung haben,

mit Hydrazinhydrat gegebenenfalls in Gegenwart eines Verdünnungsmittels wie beispielsweise Ethanol bei Temperaturen zwischen 30 ° C und 80 ° C umsetzt.

Dabei ist es auch möglich und gegebenenfalls von Vorteil, die Umsetzung der Phenacylaminderivate der Formel (Va) mit Formaldehyd und die anschließende Umsetzung der so erhältlichen Phenacyl-Enamin-Derivate der Formel (VIII) mit Hydrazinhydrat in einem Reaktionsschritt in einem sogenannten "Eintopfverfahren" durchzuführen.

Phenacylaminderivate der Formeln (V) bzw. (Va) sind bekannt oder erhältlich in Analogie zu bekannten Verfahren (vgl. z.B. Chem. Ber. 122, 295-300 [1989]; J. Med. Chem. 29, 333-341 [1986]; J. Amer. Chem. Soc. 98, 3621-3627 [1976]; Tetrahedron 31, 2145-2149 [1975]; Zh. Org. Khim. 10, 2429-2436 [1974]; J. Org. Chem. 55, 3658-3660 [1990]; Chem. Ind. 1975, 177; Synthesis 1990, 520).

Dimethylmethylenimmoniumchlorid der Formel (VI) ist bekannt (vgl. z.B. J. Org. Chem. 47, 2940-2944 [1982]; Bull. Soc. Chim. Fr. 1970, 2707-2711).

Phenacyldiaminderivate der Formel (VII) sind teilweise bekannt (vgl. z.B. US 2980674 [1957] bzw. CA 56: 3419 [1962]).

Phenacyl-Enamin-Derivate der Formel (VIII) sind bekannt oder erhältlich in Analogie zu bekannten Verfahren (vgl. z.B. Chem. Ber. 93, 387-391 [1960]; Sankyo Kenkyusho Nempo 15, 36 [1963] bzw. Chem. Abstr. 60: 11876d [1964]; J. Med. Chem. 30, 1497-1502 [1987]; JP 61-053239; US 4277420; Chem. Abstr. 87: 67928y; Khim.-Farm. Zh. 9, 21-24 [1975] bzw. CA 83: 58351v; Collect. Czech. Commun. 19, 317-328 [1954]; Ann. Chim. 46, 267-269 [1956]; J. Chem. Soc. 1953, 4066-4073; Zh. Obshch. Khim. 30, 3714 [1960]; Pharm. Chem. J. 9, 301-304 [1975]).

Die zur Durchführung des erfindungsgemäßen Verfahrens (a) als Ausgangsstoffe benötigten Isocyanate sind durch die Formel (III) allgemein definiert. In dieser Formel (III) steht $R^6$ vorzugsweise für diejenigen Reste, die bereits im Zusammenhang mit der Beschreibung der erfindungsgemäßen Verbindungen der Formel (I) genannt wurden. Die Isocyanate der Formel (III) sind allgemein bekannte Verbindungen der organischen Chemie.

Die zur Durchführung des erfindungsgemäßen Verfahrens (b) als Ausgangsstoffe benötigten Carbaminsäurechloride sind durch die Formel (IV) allgemein definiert. In dieser Formel (IV) steht $R^6$ für diejenigen Reste, die bereits im Zusammenhang mit der Beschreibung der erfindungsgemäßen Stoffe der Formel (I) für diese Substituenten genannt wurden.

Die Carbaminsäurechloride der Formel (IV) sind allgemein bekannt oder erhältlich mit Hilfe bekannter Verfahren (vgl. z.B. JP 57-108057 bzw. Chem. Abstr. 98: 53440y; JP-A 50-089344 bzw. Chem. Abstr. 83: 205987n; DE-A 2429523; AU-A 491880 bzw. Chem. Abstr. 89: 163572q).

Als Verdünnungsmittel zur Durchführung der erfindungsgemäßen Verfahren (a) und (b) kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether wie Diethylether, Diisopropylether, Dioxan, Tetrahydrofuran oder Ethylenglykoldimethyl- oder -diethylether; Ketone wie Aceton oder Butanon oder Methyl-isobutyl-keton; Nitrile wie Acetonitril, Propionitril oder Benzonitril; Amide wie N,N-Dimethylformamid, N,N-Dimethylacetamid, N-Methylformanilid, N-Methylpyrrolidon oder Hexamethylphosphorsäuretriamid, Ester wie Essigsäuremethylester oder Essigsäureethylester oder Sulfoxide wie Dimethylsulfoxid.

Die erfindungsgemäßen Verfahren (a) und (b) können vorzugsweise in Gegenwart eines geeigneten Reaktionshilfsmittels durchgeführt werden. Als solche kommen alle üblichen anorganischen oder organischen Basen in Frage. Hierzu gehören beispielsweise Erdalkali- oder Alkalimetallhydroxide wie Natriumhydroxid, Calciumhydroxid, Kaliumhydroxid oder auch Ammoniumhydroxid, Alkalimetallcarbonate wie Natriumcarbonat, Kaliumcarbonat, Kaliumhydrogencarbonat, Natriumhydrogencarbonat oder Ammoniumcarbonat, Alkali- oder Erdalkalimetallacetate wie Natriumacetat, Kaliumacetat, Calciumacetat oder Ammoniumacetat sowie tertiäre Amine wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren (a) und (b) in einem größeren Bereich variiert werden. Im allgemeinen arbeitet man bei Temperaturen zwischen 0 °C und 120 °C, vorzugsweise bei Temperaturen zwischen 20 °C und 80 °C.

Die erfindungsgemäßen Verfahren (a) und (b) werden üblicherweise unter Normaldruck durchgeführt. Es ist jedoch auch möglich unter erhöhtem oder vermindertem Druck zu arbeiten.

Zur Durchführung des erfindungsgemäßen Verfahrens (a) setzt man pro Mol an in 1-Stellung unsubstituiertem Pyrazolin der Formel (II) im allgemeinen 1,0 bis 2,5 Mol, vorzugsweise 1,0 bis 1,5 Mol an Isocyanat der Formel (III) und gegebenenfalls 0,01 bis 2,0 Mol, vorzugsweise 0,1 bis 1,2 Mol an als Reaktionshilfsmittel verwendeter Base ein.
Die Reaktionsdurchführung, Aufarbeitung und Isolierung der Reaktionsprodukte erfolgt nach allgemein üblichen Methoden (vgl. hierzu die Herstellungsbeispiele).

Zur Durchführung des erfindungsgemäßen Verfahrens (b) setzt man pro Mol an in 1-Stellung unsubstituiertem Pyrazolin der Formel (II) im allgemeinen 1,0 bis 2,5 Mol, vorzugsweise 1,0 bis 1,5 Mol an Carbaminsäurechlorid der Formel (IV) und gegebenenfalls 0,01 bis 2,0 Mol, vorzugsweise 0,1 bis 1,2 Mol an als Reaktionshilfsmittel verwendeter Base ein.
Die Reaktionsdurchführung, Aufarbeitung und Isolierung der Reaktionsprodukte erfolgt nach allgemein üblichen Methoden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber;
aus der Ordnung der Diplopoda z.B. Blaniulus guttulatus;
aus der Ordnung der Chilopoda z.B. Geophilus carpophagus, Scutigera spec;
aus der Ordnung der Symphyla z.B. Scutigerella immaculata;
aus der Ordnung der Thysanura z.B. Lepisma saccharina;
aus der Ordnung der Collembola z.B. Onychiurus armatus;
aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria;
aus der Ordnung der Dermaptera z.B. Forficula auricularia;
aus der Ordnung der Isoptera z.B. Reticulitermes spp.;
aus der Ordnung der Anoplura z.B. Phylloxera vastatrix, Pemphigus spp., Pediculus humanus corporis, Haematopinus spp., Linognathus spp;
aus der Ordnung der Mallophaga z.B. Trichodectes spp., Damalinea spp;
aus der Ordnung der Thysanoptera z.B. Hercinothrips femoralis, Thrips tabaci;
aus der Ordnung der Heteroptera z.B. Eurygaster spp., Dysdercus intermedius, Piesma quadrata, Cimex lectularius, Rhodnius prolixus, Triatoma spp;
aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp;
aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana;
aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica;
aus der Ordnung der Hymenoptera z.B. Diprion spp., Hoplocampa spp., Lasius spp., Monomorium pharaonis, Vespa spp;
aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa;
aus der Ordnung der Siphonaptera z.B. Xenopsylla cheopis, Ceratophyllus spp..
Aus der Ordnung der Arachnida z.B. Scorpio maurus, Latrodectus mactans;
aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Dabei lassen sich die erfindungsgemäßen Wirkstoffe mit besonders gutem Erfolg zur Bekämpfung von pflanzenschädigenden Insekten wie beispielsweise gegen die Larven der Meeretttichblattkäfer (Phaedon cochleariae) oder gegen die Raupen der Kohlschabe (Plutella maculipennis) einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Herstellung und Anwendung der erfindungsgemäßen Wirkstoffe geht aus den nachfolgenden Beispielen hervor:

Herstellungsbeispiele

Beispiel 1

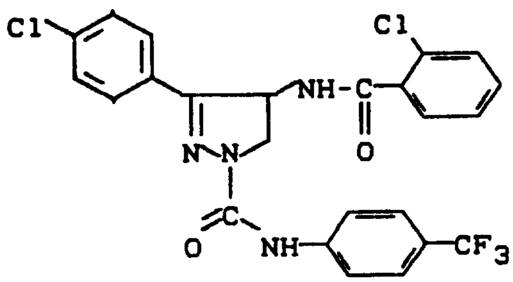

(Verfahren (a))

Zu 2 g (0,00598 Mol) 3-(4-Chlorphenyl)-4-[(2-chlorphenyl)-carbonylamino]-4,5-dihydropyrazol in 50 ml Dichlormethan gibt man bei Raumtemperatur tropfenweise unter Rühren 1,2 g (0,00642 Mol) 4-Trifluormethylphenylisocyanat und erwärmt für eine Stunde auf Rückflußtemperatur.

Zur Aufarbeitung wird die Reaktionsmischung abgekühlt, ausgefallener Feststoff abgesaugt und getrocknet.

Man erhält 1,6 g (51 % d. Theor.) 3-(4-Chlorphenyl)-4-[(2-chlorphenyl)-Carbonylamino]-1-[(4-trifluormethylphenyl)-aminocarbonyl]-4,5-dihydropyrazol vom Schmelzpunkt > 200 °C.

$^1$H-NMR (DMSO-$d_6$/Tetramethylsilan):

$\delta$ = 3,96(dd); 4,29(t); 5,95(m); 9,33(d); 9,53(s) ppm.

In entsprechender Weise und gemäß den allgemeinen Angaben zur Herstellung erhält man die folgenden substituierten Pyrazoline der allgemeinen Formel (I):

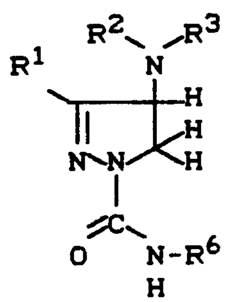

(I)

| Bsp. | $R^1$ | $R^2$ | $R^3$ | $R^6$ |
|---|---|---|---|---|
| 2 | 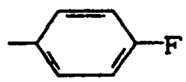 | 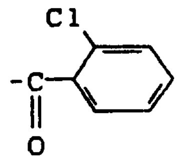 | H | 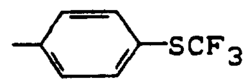 |
| 3 | 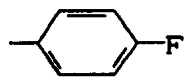 | 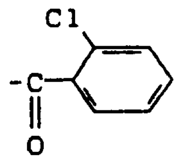 | H | 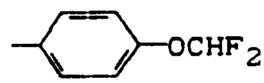 |
| 4 | 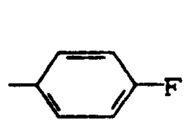 | 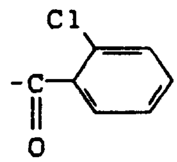 | H | 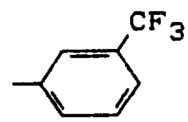 |
| 5 | 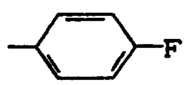 | 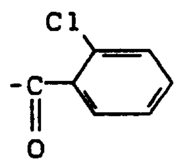 | H | 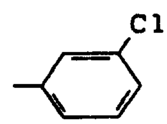 |
| 6 | 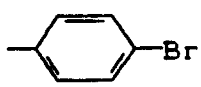 | 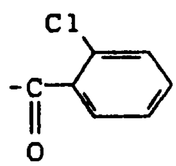 | H | 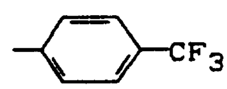 |

| Bsp. | $R^1$ | $R^2$ | $R^3$ | $R^6$ |
|---|---|---|---|---|
| 7 | 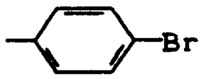 | 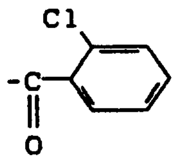 | H | 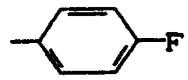 |
| 8 | 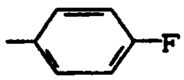 | 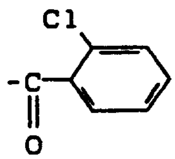 | H | 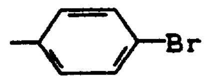 |
| 9 | 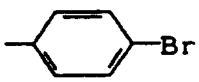 | 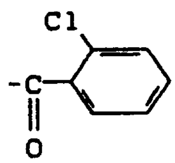 | H | 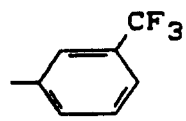 |
| 10 | 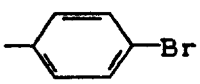 | 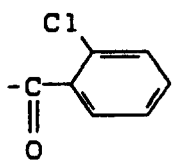 | H | 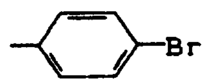 |
| 11 | 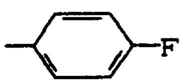 | 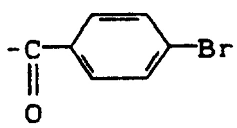 | H | 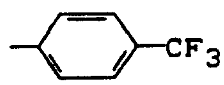 |
| 12 | 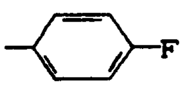 | 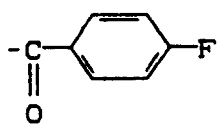 | H | 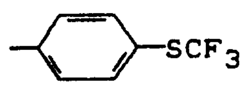 |
| 13 | 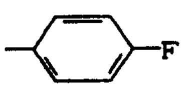 | 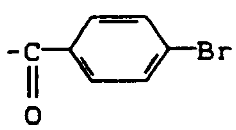 | H | 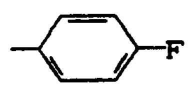 |
| 14 | 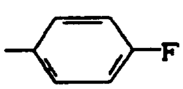 | 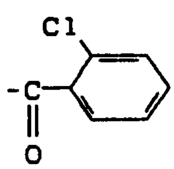 | H | 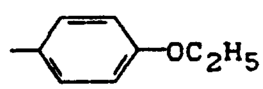 |

| Bsp. | $R^1$ | $R^2$ | $R^3$ | $R^6$ |
|---|---|---|---|---|
| 15 | 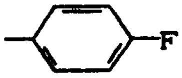 | 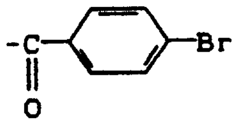 | H | 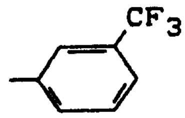 |
| 16 | 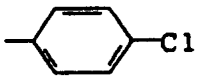 | 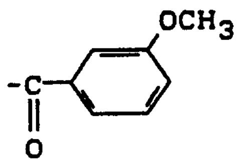 | H | 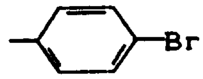 |
| 17 | 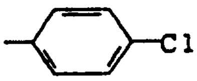 | 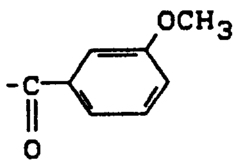 | H | 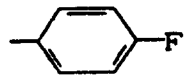 |
| 18 | 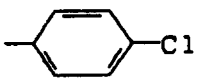 | 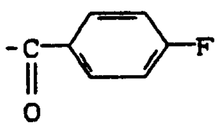 | H | 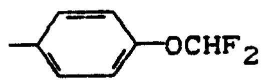 |
| 19 | 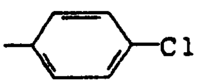 | 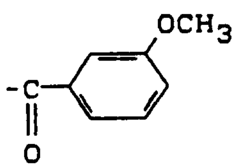 | H | 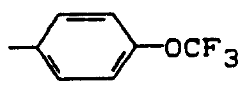 |
| 20 | 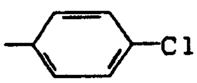 | 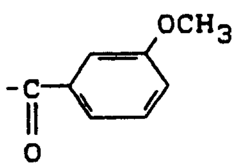 | H | 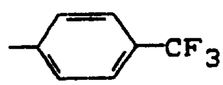 |
| 21 | 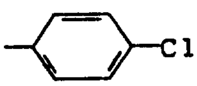 | 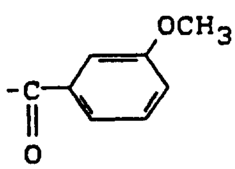 | H | 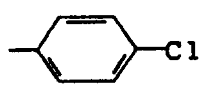 |
| 22 | 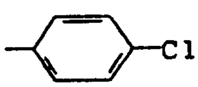 | 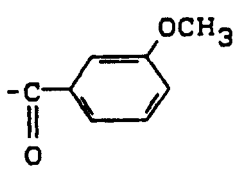 | H | 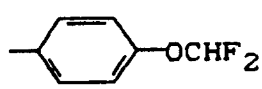 |

| Bsp. | $R^1$ | $R^2$ | $R^3$ | $R^6$ |
| --- | --- | --- | --- | --- |
| 23 | 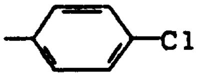 | 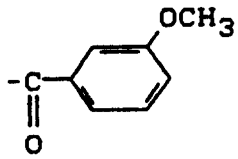 | H | 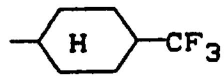 |
| 24 | 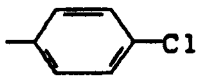 | 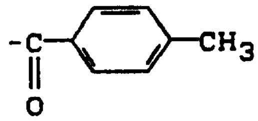 | H | 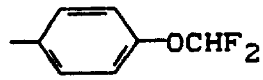 |
| 25 | 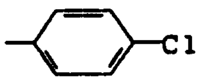 | 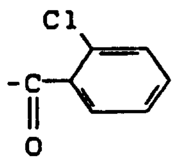 | H | 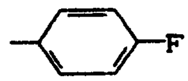 |
| 26 | 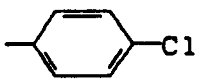 | 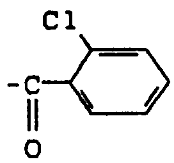 | H | 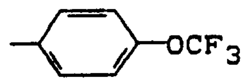 |
| 27 | 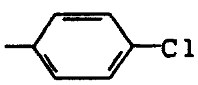 | 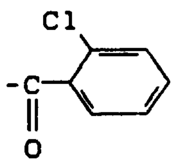 | H | 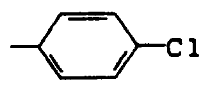 |
| 28 | 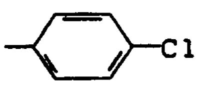 | 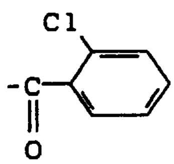 | H | 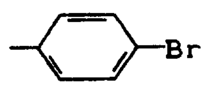 |
| 29 | 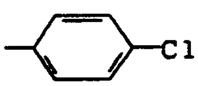 | 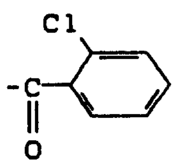 | H | 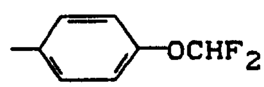 |
| 30 | 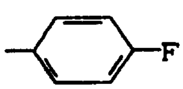 | 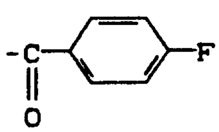 | H | 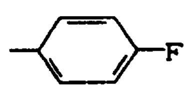 |

| Bsp. | $R^1$ | $R^2$ | $R^3$ | $R^6$ |
|---|---|---|---|---|
| 31 | 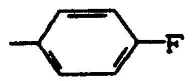 | 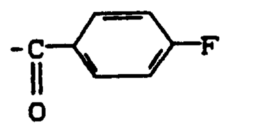 | H | 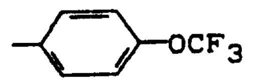 |
| 32 | 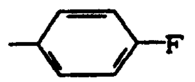 | 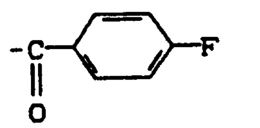 | H | 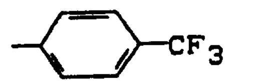 |
| 33 | 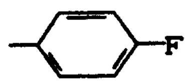 | 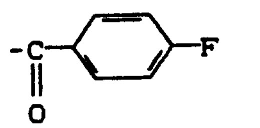 | H | 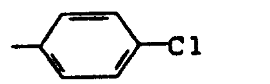 |
| 34 | 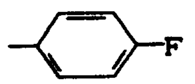 | 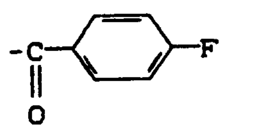 | H | 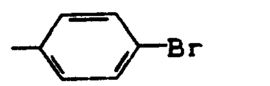 |
| 35 | 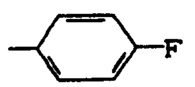 | 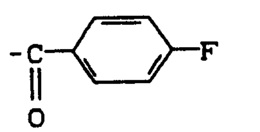 | H | 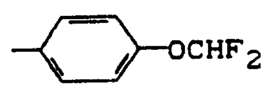 |
| 36 | 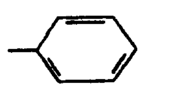 | 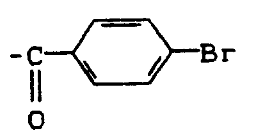 | H | 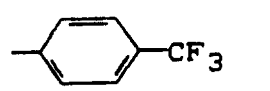 |
| 37 | 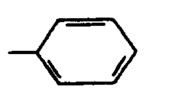 | 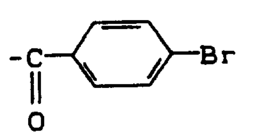 | H | 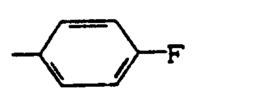 |
| 38 | 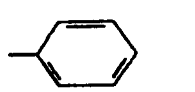 | 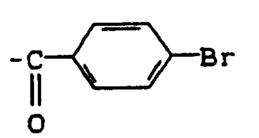 | H | 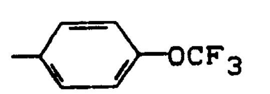 |
| 39 | 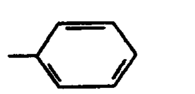 | 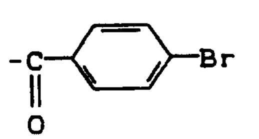 | H | 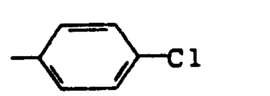 |

| Bsp. | $R^1$ | $R^2$ | $R^3$ | $R^6$ |
|---|---|---|---|---|
| 40 | 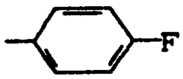 | 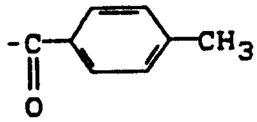 | H | 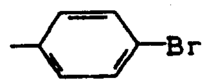 |
| 41 | 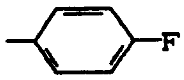 | 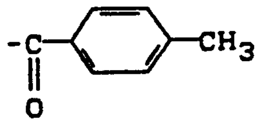 | H | 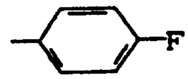 |
| 42 | 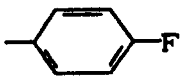 | 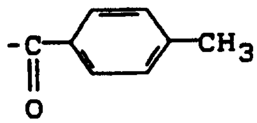 | H | 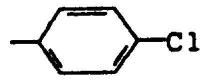 |
| 43 | 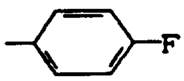 | 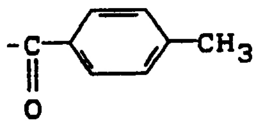 | H | 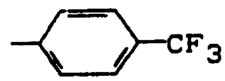 |
| 44 | 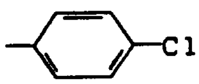 | 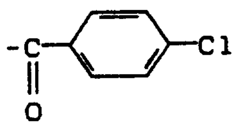 | H | 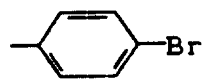 |
| 45 | 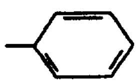 | 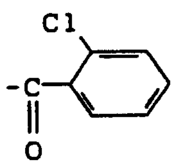 | H | 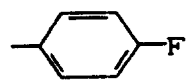 |
| 46 | 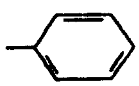 | 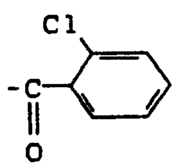 | H | 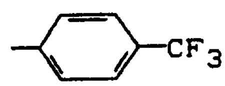 |
| 47 | 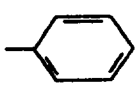 | 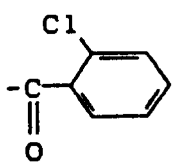 | H | 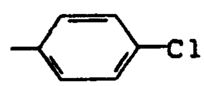 |

| Bsp. | $R^1$ | $R^2$ | $R^3$ | $R^6$ |
|---|---|---|---|---|
| 48 | 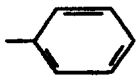 | 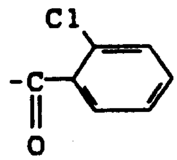 | H | 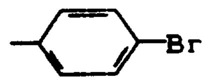 |
| 49 | 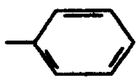 | 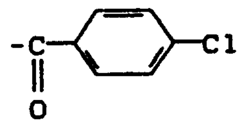 | H | 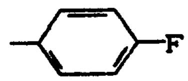 |
| 50 | 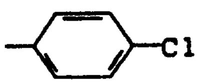 | 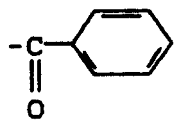 | H | 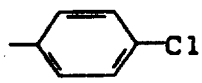 |
| 51 | 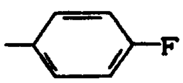 | 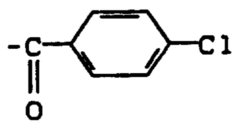 | H | 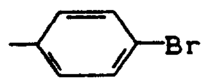 |
| 52 | 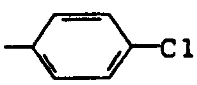 | 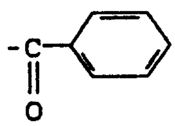 | H | 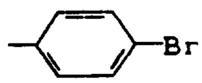 |
| 53 | 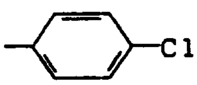 | 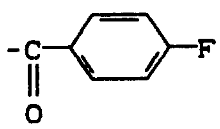 | H | 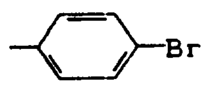 |
| 54 | 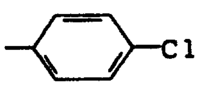 | 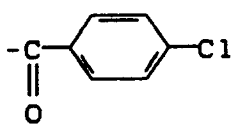 | H | 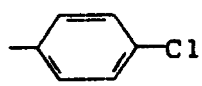 |
| 55 | 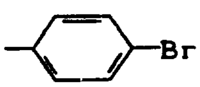 | 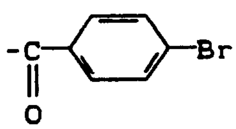 | H | 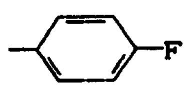 |
| 56 | 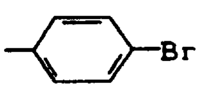 | 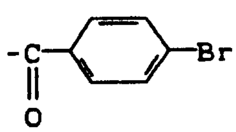 | H | 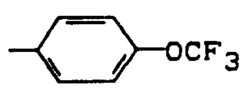 |

| Bsp. | $R^1$ | $R^2$ | $R^3$ | $R^6$ |
|---|---|---|---|---|
| 57 | 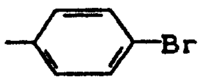 | 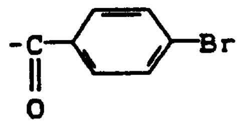 | H | 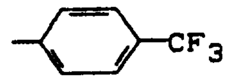 |
| 58 | 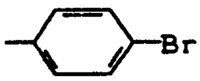 | 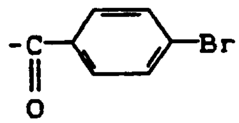 | H | 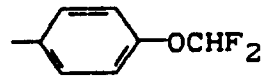 |
| 59 | 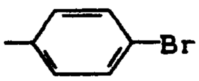 | 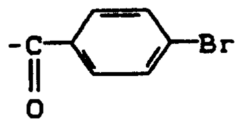 | H | 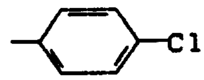 |
| 60 | 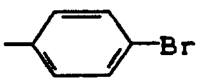 | 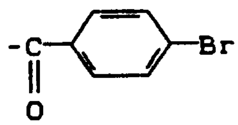 | H | 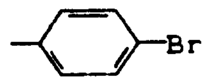 |
| 61 | 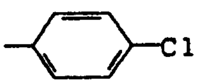 | 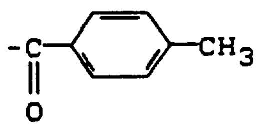 | H | 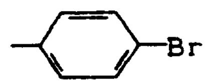 |
| 62 | 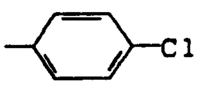 | 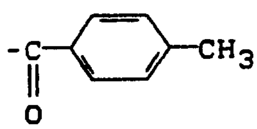 | H | 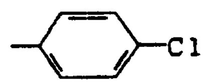 |
| 63 | 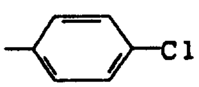 | 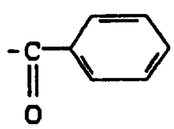 | H | 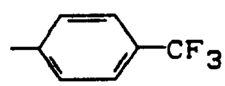 |
| 64 | 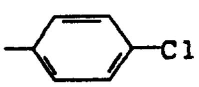 | 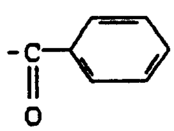 | H | 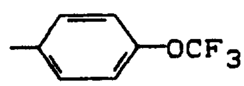 |
| 65 | 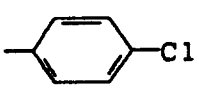 | 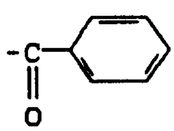 | H | 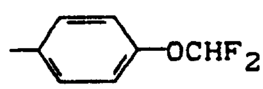 |

| Bsp. | $R^1$ | $R^2$ | $R^3$ | $R^6$ |
|---|---|---|---|---|
| 66 | 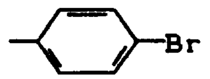 | 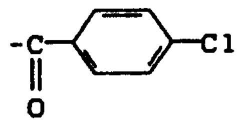 | H | 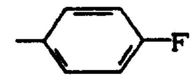 |
| 67 | 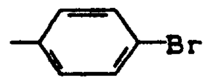 | 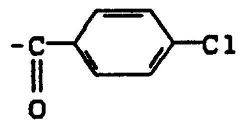 | H | 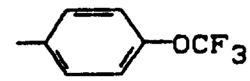 |
| 68 | 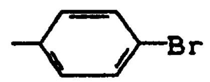 | 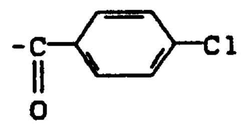 | H | 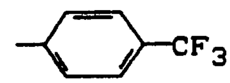 |
| 69 | 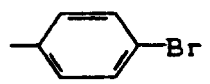 | 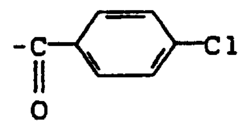 | H | 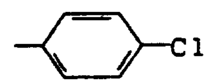 |
| 70 | 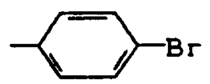 | 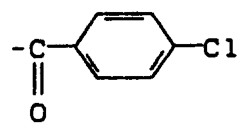 | H | 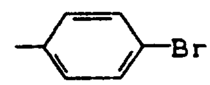 |
| 71 | 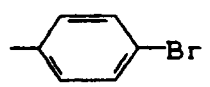 | 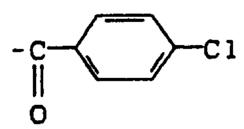 | H | 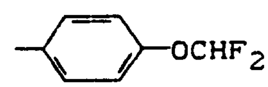 |
| 72 | 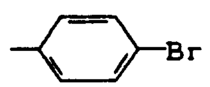 | 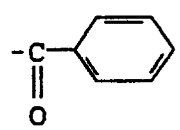 | H | 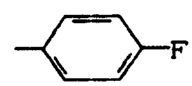 |
| 73 | 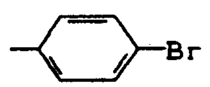 | 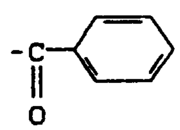 | H | 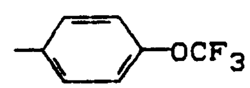 |
| 74 | 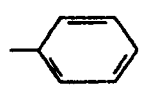 | 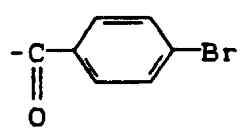 | H | 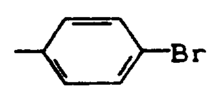 |

| Bsp. | $R^1$ | $R^2$ | $R^3$ | $R^6$ |
|---|---|---|---|---|
| 75 | 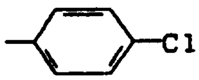 | 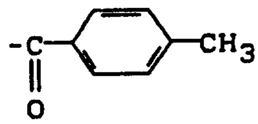 | H | 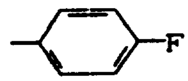 |
| 76 | 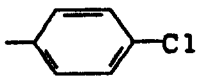 | 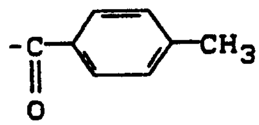 | H | 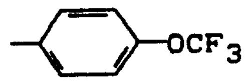 |
| 77 | 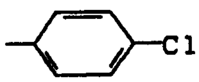 | 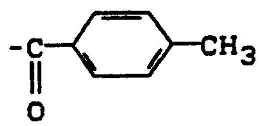 | H | 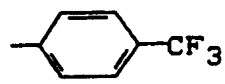 |
| 78 | 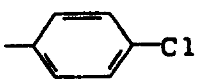 | 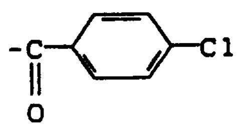 | H | 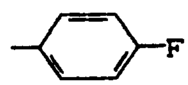 |
| 79 | 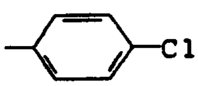 | 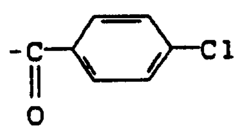 | H | 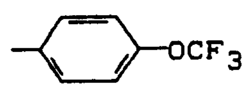 |
| 80 | 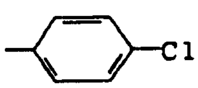 | 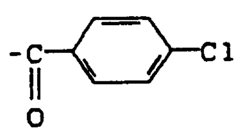 | H | 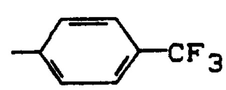 |
| 81 | 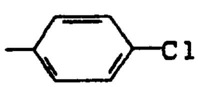 | 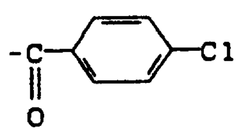 | H | 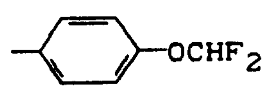 |
| 82 | 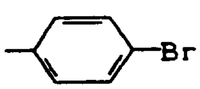 | 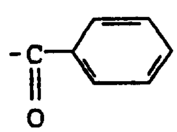 | H | 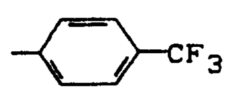 |
| 83 | 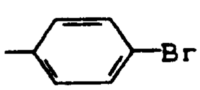 | 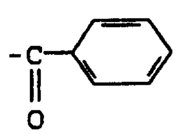 | H | 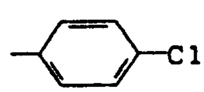 |

| Bsp. | $R^1$ | $R^2$ | $R^3$ | $R^6$ |
|---|---|---|---|---|
| 84 | 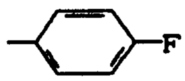 | 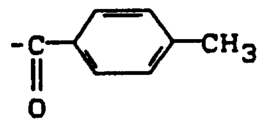 | H | 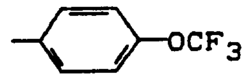 |
| 85 | 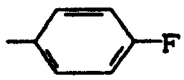 | 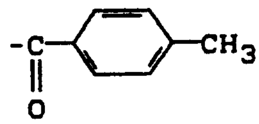 | H | 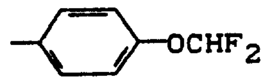 |
| 86 | 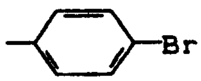 | 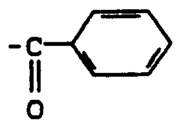 | H | 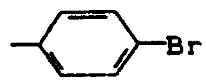 |
| 87 | 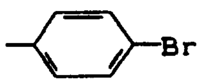 | 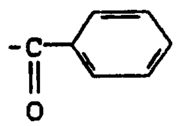 | H | 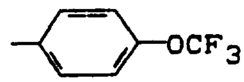 |
| 88 | 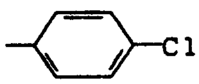 | 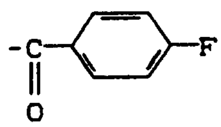 | H | 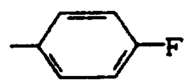 |
| 89 | 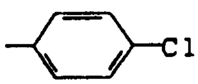 | 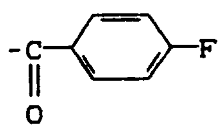 | H | 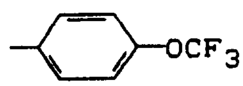 |
| 90 | 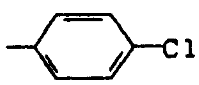 | 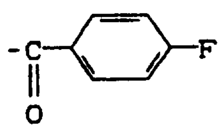 | H | 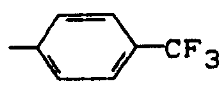 |
| 91 | 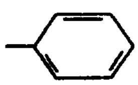 | 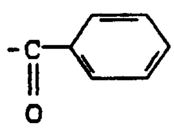 | H | 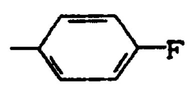 |
| 92 | 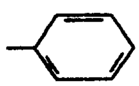 | 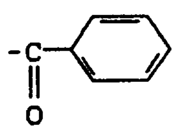 | H | 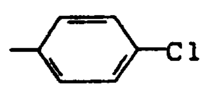 |

| Bsp. | $R^1$ | $R^2$ | $R^3$ | $R^6$ |
|---|---|---|---|---|
| 93 | 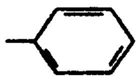 | 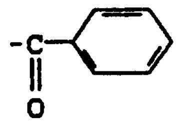 | H | 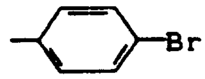 |
| 94 | 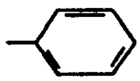 | 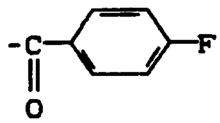 | H | 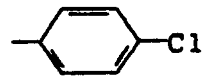 |
| 95 | 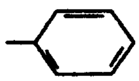 | 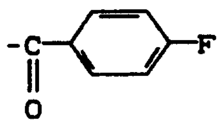 | H | 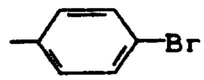 |
| 96 | 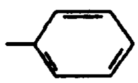 | 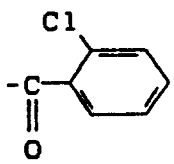 | H | 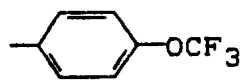 |
| 97 | 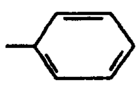 | 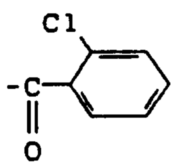 | H | 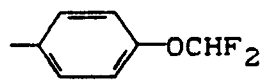 |
| 98 | 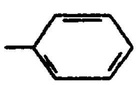 | 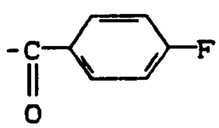 | H | 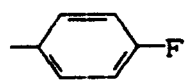 |
| 99 | 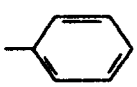 | 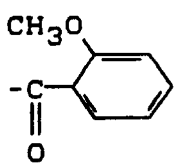 | H | 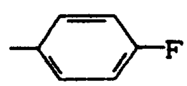 |
| 100 | 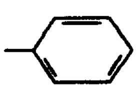 | 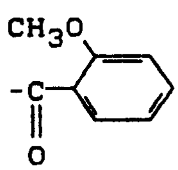 | H | 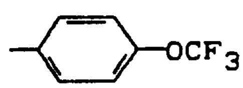 |

| Bsp. | $R^1$ | $R^2$ | $R^3$ | $R^6$ |
|---|---|---|---|---|
| 101 | 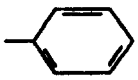 | 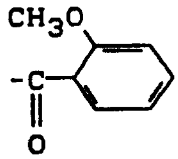 | H | 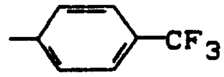 |
| 102 | 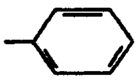 | 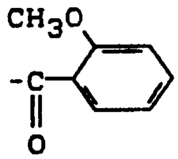 | H | 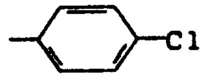 |
| 103 | 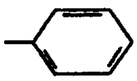 | 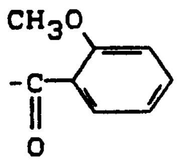 | H | 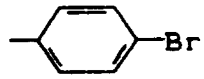 |
| 104 | 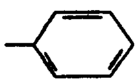 | 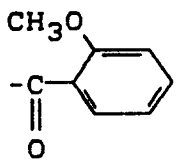 | H | 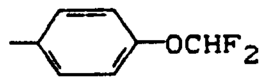 |
| 105 | 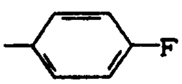 | 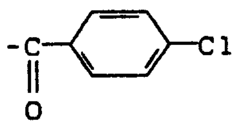 | H | 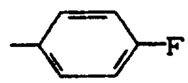 |
| 106 | 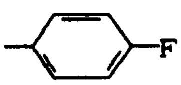 | 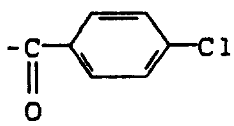 | H | 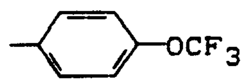 |
| 107 | 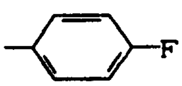 | 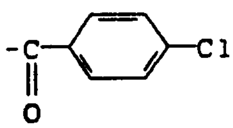 | H | 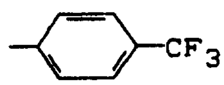 |
| 108 | 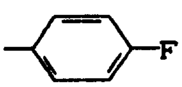 | 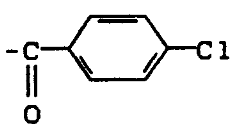 | H | 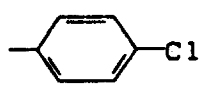 |

| Bsp. | $R^1$ | $R^2$ | $R^3$ | $R^6$ |
|---|---|---|---|---|
| 109 | 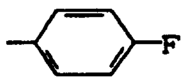 | 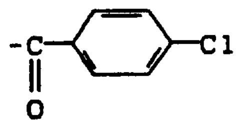 | H | 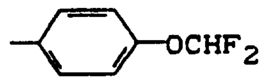 |
| 110 | 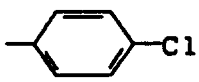 | 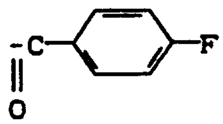 | H | 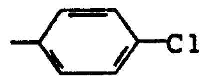 |
| 111 | 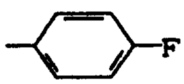 | 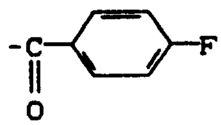 | H | 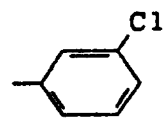 |
| 112 | 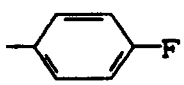 | 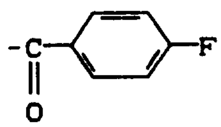 | H | 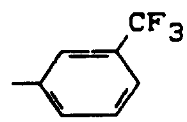 |
| 113 | 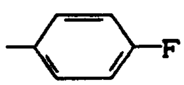 | 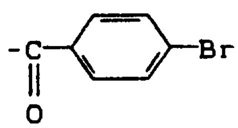 | H | 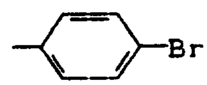 |
| 114 | 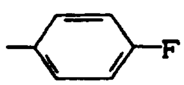 | 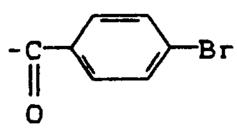 | H | 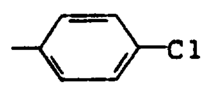 |
| 115 | 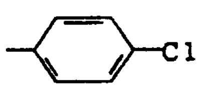 | 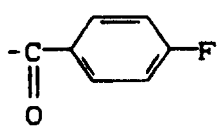 | H | 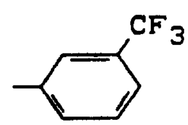 |
| 116 | 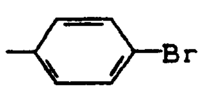 | 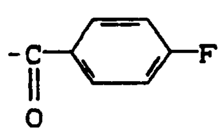 | H | 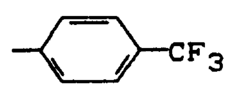 |
| 117 | 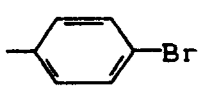 | 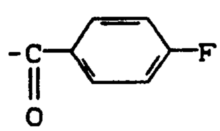 | H | 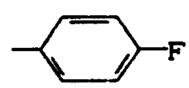 |

| Bsp. | $R^1$ | $R^2$ | $R^3$ | $R^6$ |
|---|---|---|---|---|
| 118 | 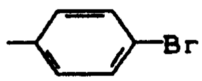 | 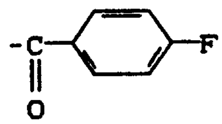 | H | 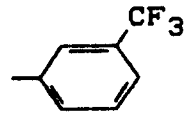 |
| 119 | 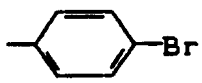 | 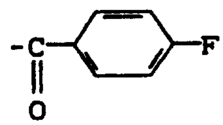 | H | 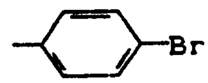 |
| 120 | 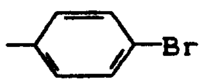 | 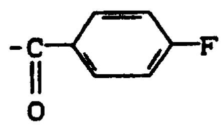 | H | 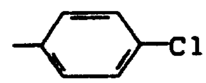 |
| 121 | 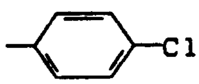 | 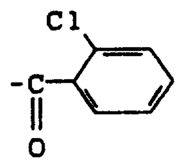 | H | 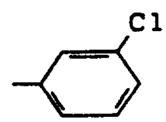 |
| 122 | 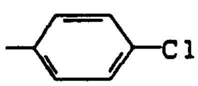 | 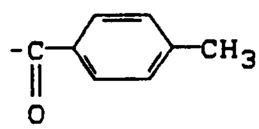 | H | 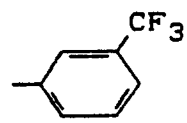 |
| 123 | 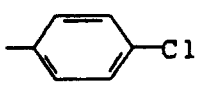 | 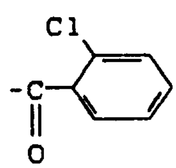 | H | 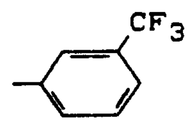 |
| 124 | 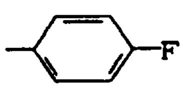 | 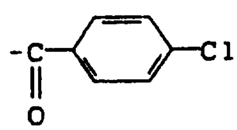 | H | 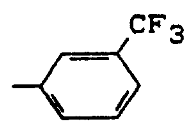 |
| 125 | 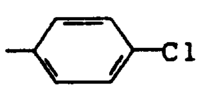 | 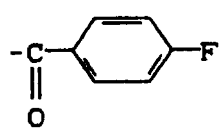 | H | 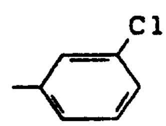 |

| Bsp. | $R^1$ | $R^2$ | $R^3$ | $R^6$ |
|---|---|---|---|---|
| 126 | 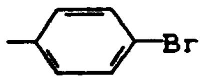 | | $-\overset{O}{\overset{\|}{C}}-(CH_2)_5-$ | 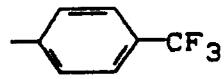 |
| 127 | 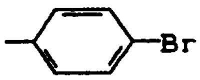 | $-\overset{O}{\overset{\|}{C}}-(CH_2)_5-$ | | 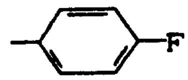 |
| 128 | 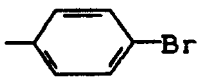 | $-\overset{O}{\overset{\|}{C}}-(CH_2)_5-$ | | 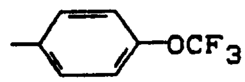 |
| 129 | 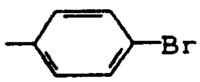 | $-\overset{O}{\overset{\|}{C}}-(CH_2)_5-$ | | 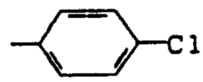 |
| 130 | 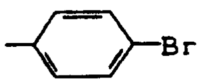 | $-\overset{O}{\overset{\|}{C}}-(CH_2)_5-$ | | 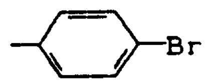 |
| 131 | 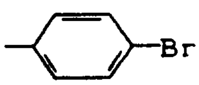 | $-\overset{O}{\overset{\|}{C}}-(CH_2)_5-$ | | 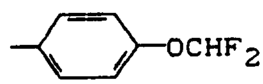 |
| 132 | 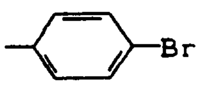 | | $-\overset{O}{\overset{\|}{C}}-(CH_2)_5-$ | 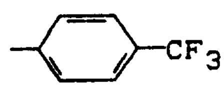 |
| 133 | 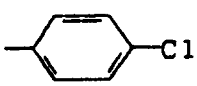 | $-\underset{O}{\underset{\|}{C}}-CH_3$ | H | 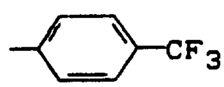 |
| 134 | 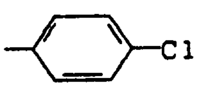 | 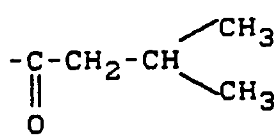 | H | 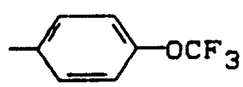 |

| Bsp. | $R^1$ | $R^2$ | $R^3$ | $R^6$ |
|---|---|---|---|---|
| 135 | 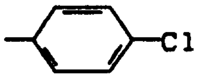 | 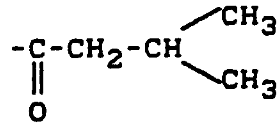 | H | 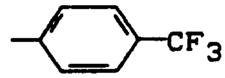 |
| 136 | 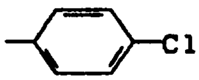 | 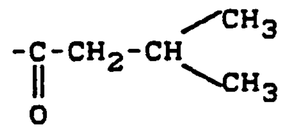 | H | 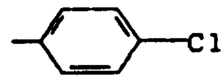 |
| 137 | 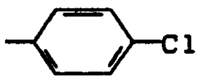 | 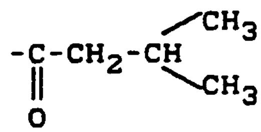 | H | 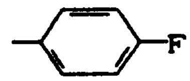 |
| 138 | 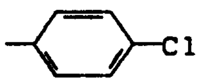 | 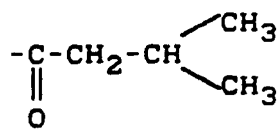 | H | 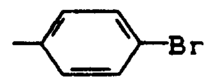 |
| 139 | 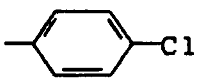 | 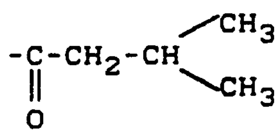 | H | 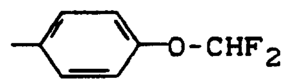 |
| 140 | 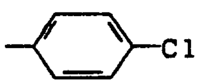 | 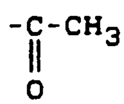 | H | 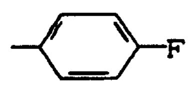 |
| 141 | 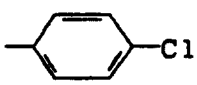 | 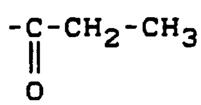 | H | 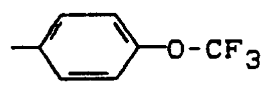 |
| 142 | 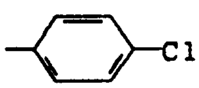 | 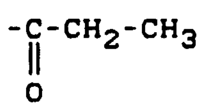 | H | 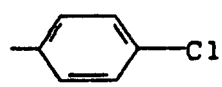 |
| 143 | 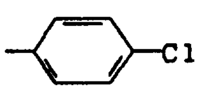 | 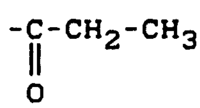 | H | 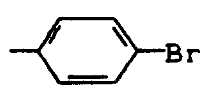 |

| Bsp. | $R^1$ | $R^2$ | $R^3$ | $R^6$ |
|---|---|---|---|---|
| 144 | 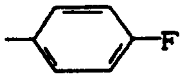 | 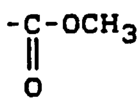 | H | 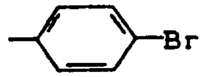 |
| 145 | 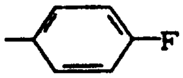 | 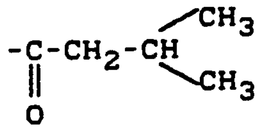 | H | 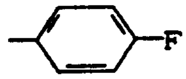 |
| 146 | 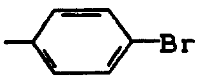 | | 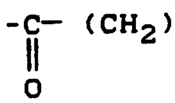 | 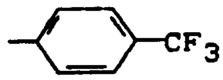 |
| 147 | 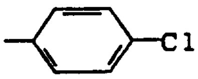 | 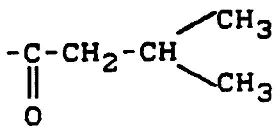 | H | 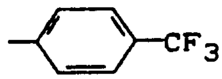 |
| 148 | 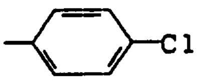 | 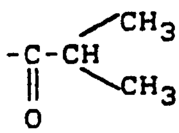 | H | 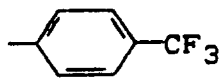 |
| 149 | 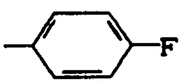 | 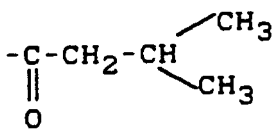 | H | 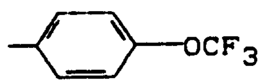 |
| 150 | 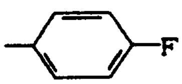 | 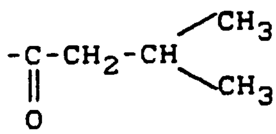 | H | 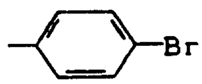 |
| 151 | 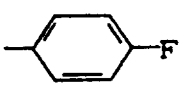 | 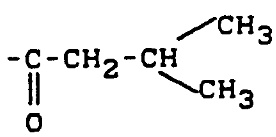 | H | 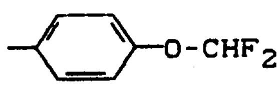 |
| 152 | 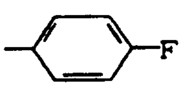 | 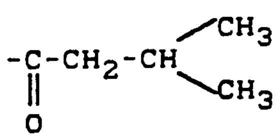 | H | 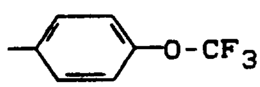 |

| Bsp. | $R^1$ | $R^2$ | $R^3$ | $R^6$ |
|---|---|---|---|---|
| 153 | 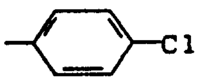 | 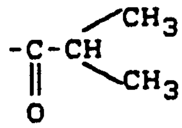 | H | 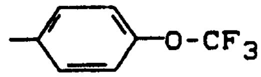 |
| 154 | 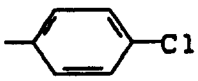 | 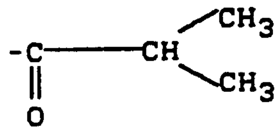 | H | 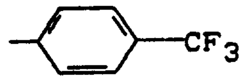 |
| 155 | 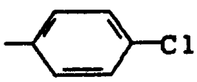 | 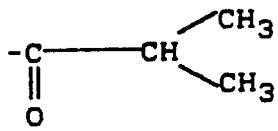 | H | 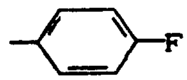 |
| 156 | 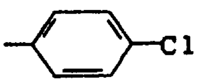 | 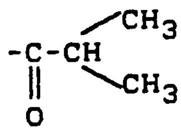 | H | 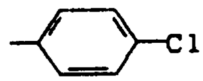 |
| 157 | 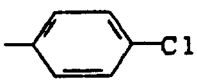 | 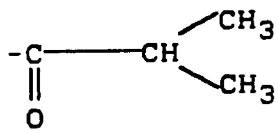 | H | 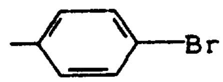 |
| 158 | 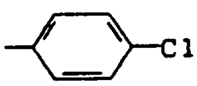 | 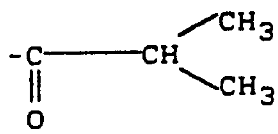 | H | 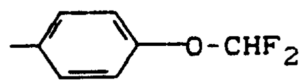 |
| 159 | 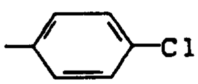 | 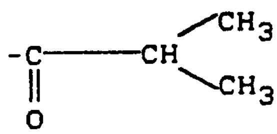 | H | 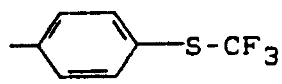 |
| 160 | 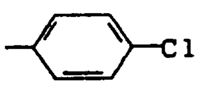 | 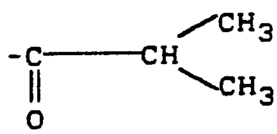 | H | 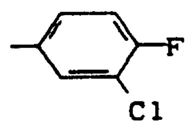 |
| 161 | 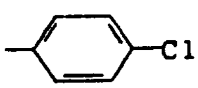 | 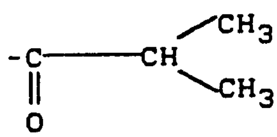 | H | 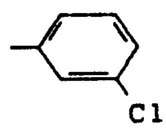 |

| Bsp. | $R^1$ | $R^2$ | $R^3$ | $R^6$ |
|---|---|---|---|---|
| 162 | 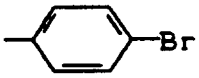 | | 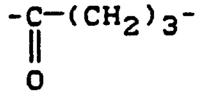 | 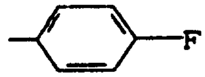 |
| 163 | 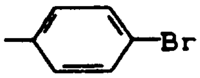 | | 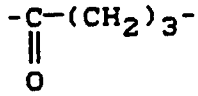 | 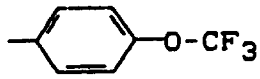 |
| 164 | 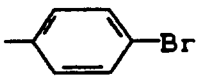 | | 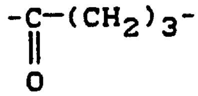 | 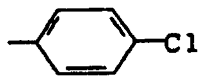 |
| 165 | 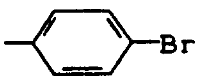 | 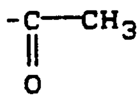 | H | 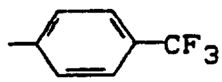 |
| 166 | 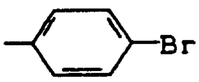 | 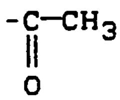 | H | 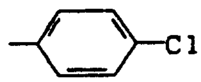 |
| 167 | 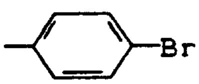 | 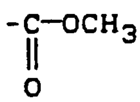 | H | 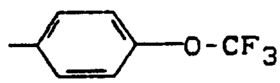 |
| 168 | 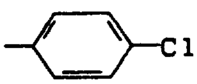 | 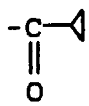 | H | 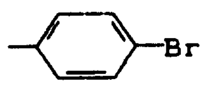 |
| 169 | 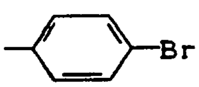 | | 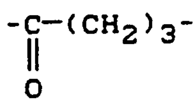 | 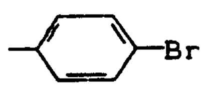 |
| 170 | 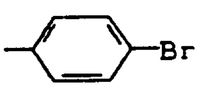 | | 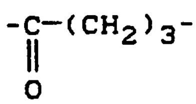 | 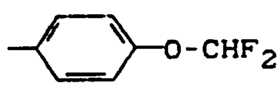 |

| Bsp. | $R^1$ | $R^2$ | $R^3$ | $R^6$ |
|---|---|---|---|---|
| 171 | 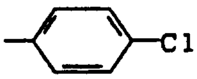 | 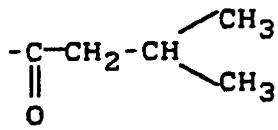 | H | 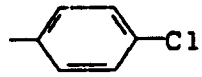 |
| 172 | 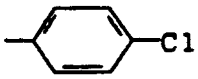 | 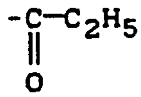 | H | 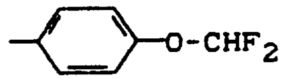 |
| 173 | 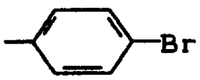 | 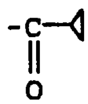 | H | 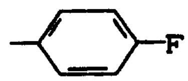 |
| 174 | 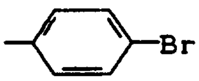 | 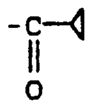 | H | 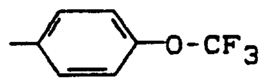 |
| 175 | 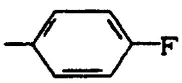 | 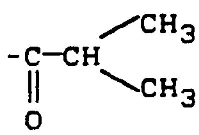 | H | 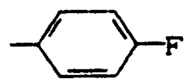 |
| 176 | 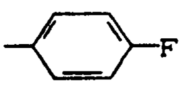 | 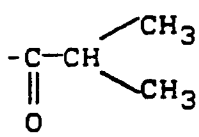 | H | 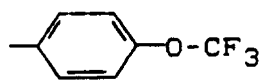 |
| 177 | 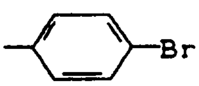 | 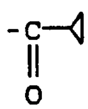 | H | 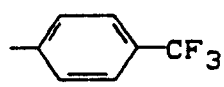 |
| 178 | 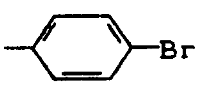 | 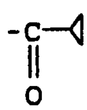 | H | 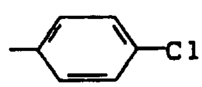 |
| 179 | 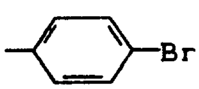 | 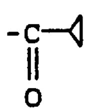 | H | 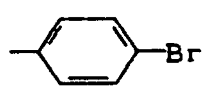 |

| Bsp. | $R^1$ | $R^2$ | $R^3$ | $R^6$ |
|---|---|---|---|---|
| 180 | 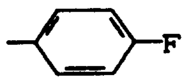 | 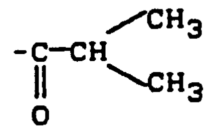 | H | 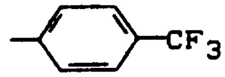 |
| 181 | 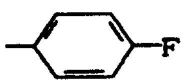 | 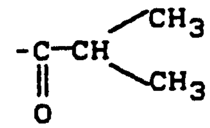 | H | 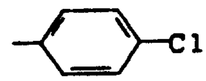 |
| 182 | 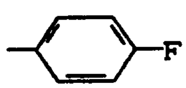 | 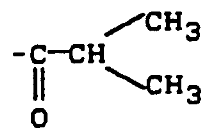 | H | 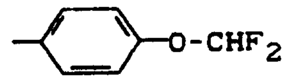 |

Herstellung der Ausgangsverbindungen:

Beispiel II-1:

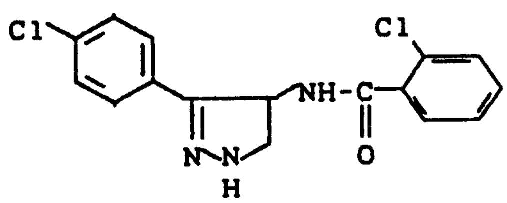

Zu 20 g (0,065 Mol) 2-Chlor-N-[2-(4-chlorphenyl)-2-oxo-ethyl]-benzamid (Herstellung vgl. z.B. J. Amer. Chem. Soc. 77, 1850 [1955]) in 100 ml Acetonitril gibt man bei Raumtemperatur unter Rühren 8,0 g (0,0856 Mol) N,N-Dimethylmethylenimmoniumchloridund erwärmt für 3 bis 5 Stunden auf Rückflußtemperatur. Wenn sich im Dünnschichtchromatogram der Reaktionsmischung keine Ausgangsverbindung mehr nachweisen läßt, gibt man 6,5 ml Hydrazinhydrat zu und erwärmt für weitere 4 bis 6 Stunden auf Rückflußtemperatur. Zur Aufarbeitung wird die Reaktionsmischung abgekühlt, ausgefallener Feststoff abgesaugt, zunächst mit Diethylether und anschließend mit Wasser gewaschen und getrocknet.

Man erhält 10,4 g (48% der Theorie) an 3-(4-Chlorphenyl)-4-[(2-chlorphenyl)-carbonylamino]-4,5-dihydropyrazol als braunen Feststoff, der ohne weitere Reinigung in die nächste Reaktion eingesetzt werden kann.

$^1$H-NMR (DMSO-$d_6$/Tetramethylsilan): $\delta$ = 5,68; 9,10 ppm

Beispiel II-2:

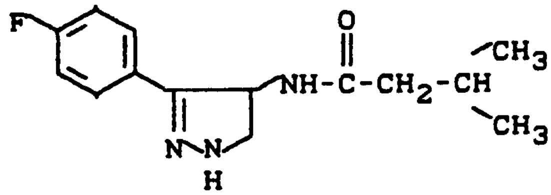

Zu 37,0 g (0,149 Mol) 4'-Fluor-2-isobutylcarbonylaminoacrylophenon in 150 ml Ethanol gibt man bei 40-50 ° C unter Rühren 29 ml Hydrazinhydrat und rührt eine weitere Stunde bei 50 ° C. Zur Aufarbeitung wird die Reaktionsmischung im Vakuum eingeengt und das dabei ausfallende Produkt abgesaugt und getrocknet.

Man erhält 37,0 g (95% der Theorie) und 3-(4-Fluorphenyl)-4-isobutylcarbonylamino-4,5-dihydropyrazol als gelben Feststoff, der ohne zusätzliche Reinigung direkt weiter umgesetzt werden kann.

Beispiel VIII-1:

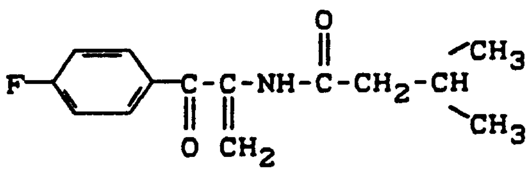

Zu 37,4 g (0,158 Mol) 2-Isobutylcarbonylamino-4'-fluoracetophenon (Herstellung vgl. z.B. DE 2947140; J. Heterocycl. Chem. 24, 297-301 [1987]) in 240 ml Methanol gibt man unter Rühren bei 60 ° C nacheinander 50 ml 37%ige wäßrige Formaldehydlösung, 1 ml Piperidin und 1 ml Eisessig, rührt anschließend eine weitere Stunde bei 60 ° C und entfernt dann das Lösungsmittel im Vakuum.

Man erhält 37,0 g (94% der Theorie) an 4'-Fluor-2-isobutylcarbonylamino-acrylophenon als Feststoff, der ohne zusätzliche Reinigung direkt weiter umgesetzt werden kann.

In entsprechender Weise erhält man die folgenden in 1-Stellung unsubstituierten Pyrazoline der allgemeinen Formel (II):

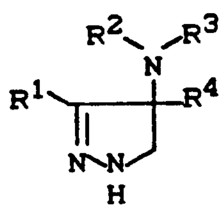

(II)

| Bsp.-Nr. | $R^1$ | $R^2$ | $R^3$ | $R^4$ | $^1H$-NMR*) |
|---|---|---|---|---|---|
| II-3 | 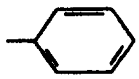 | 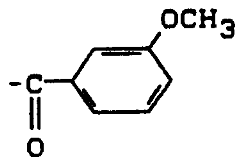 | H | H | 5,80; 9,02 |
| II-4 | 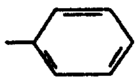 | 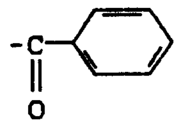 | H | H | 5,82; 9,09 |
| II-5 | 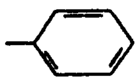 | 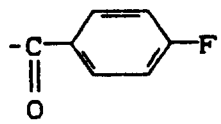 | H | H | 5,77; 9,06 |
| II-6 | 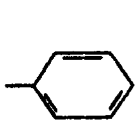 | 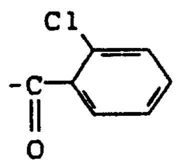 | H | H | 5,70; 9,09 |
| II-7 | 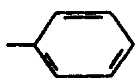 | 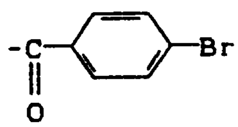 | H | H | 5,75; 9,13 |
| II-8 | 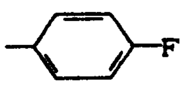 | 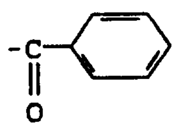 | H | H | 5,84; 9,08 |
| II-9 | 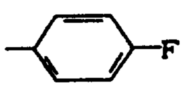 | 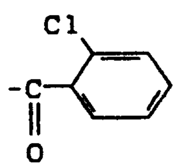 | H | H | 5,69; 9,08 |

| Bsp.-Nr. | $R^1$ | $R^2$ | $R^3$ | $R^4$ | $^{1}H$-NMR*) |
|---|---|---|---|---|---|
| II-10 | 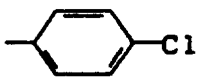 | 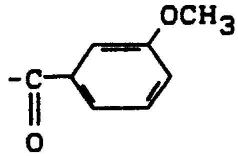 | H | H | 5,77; 9,01 |
| II-11 | 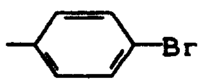 | 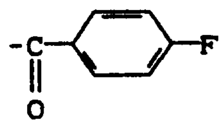 | H | H | 5,74; 9,03 |
| II-12 | 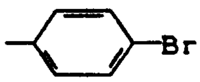 | 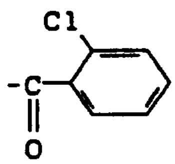 | H | H | 5,67; 9,07 |
| II-13 | 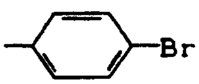 | 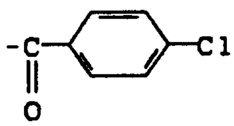 | H | H | 5,74; 9,09 |
| II-14 | 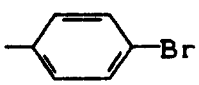 | 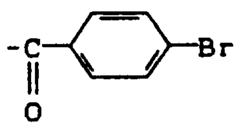 | H | H | 5,74; 9,11 |
| II-15 | 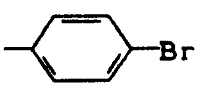 | 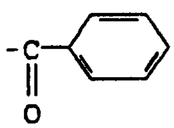 | H | H | 5,77; 9,01 |
| II-16 | 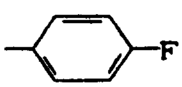 | 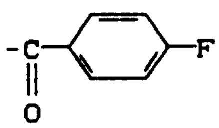 | H | H | 5,76; 9,06 |
| II-17 | 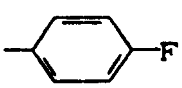 | 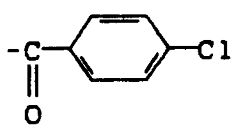 | H | H | 5,75; 9,11 |
| II-18 | 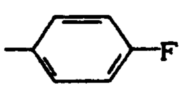 | 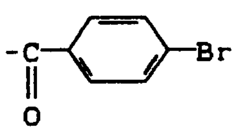 | H | H | 5,74; 9,12 |
| II-19 | 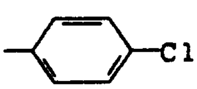 | 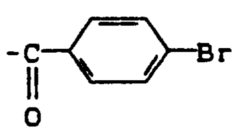 | H | H | 5,77; 8,96 |

| Bsp.-Nr. | $R^1$ | $R^2$ | $R^3$ | $R^4$ | $^1H$-NMR*) |
|---|---|---|---|---|---|
| II-20 | 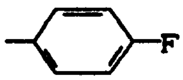 | 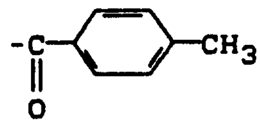 | H | H | 5,78; 8,93 |
| II-21 | 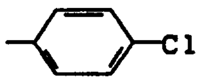 | 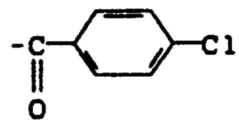 | H | H | 5,76; 9,11 |
| II-22 | 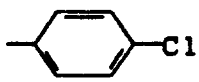 | 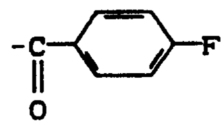 | H | H | 5,75; 9,04 |
| II-23 | 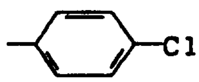 | 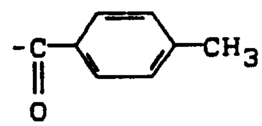 | H | H | 5,75; 8,92 |
| II-24 | 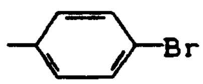 | 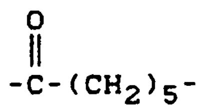 | | H | 6,12; 7,68 |
| II-25 | 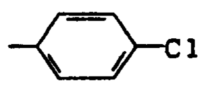 | 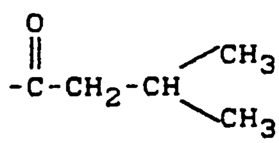 | H | H | 5,49; 8,40 |
| II-26 | 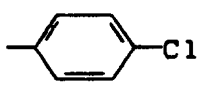 | 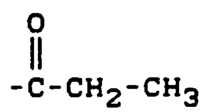 | H | H | 5,37; 8,37 |
| II-27 | 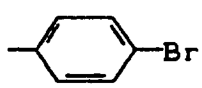 | 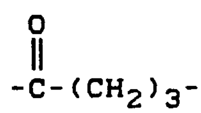 | | H | 5,86 |

| Bsp.-Nr. | $R^1$ | $R^2$ | $R^3$ | $R^4$ | $^1$H-NMR*) |
|---|---|---|---|---|---|
| II-28 | 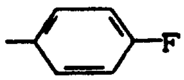 | 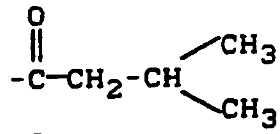 | H | H | 5,50; 8,40 |
| II-29 | 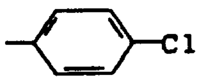 | 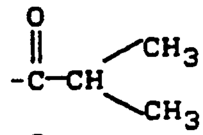 | H | H | 5,47; 8,35 |
| II-30 | 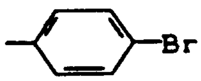 | 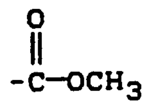 | H | H | 5,23; 7,79 |
| II-31 | 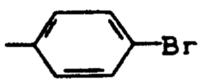 | 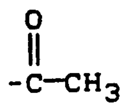 | H | H | 5,44; 8,45 |
| II-32 | 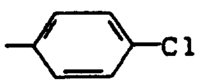 | 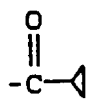 | H | H | 5,48; 8,67 |
| II-33 | 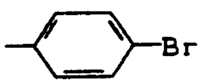 | 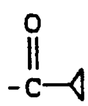 | H | H | 5,48; 8,64 |
| II-34 | 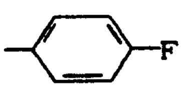 | 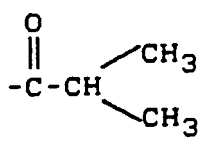 | H | H | 5,49; 8,33 |

Anwendungsbeispiele:

In den folgenden Anwendungsbeispielen wurde die nachstehend aufgeführte Verbindung als Vergleichssubstanz eingesetzt:

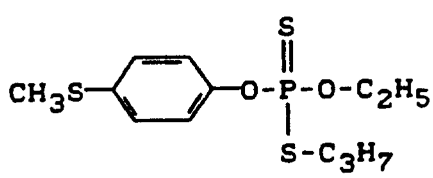

O-(4-Methylthiophenyl)-O-ethyl-S-(n-propyl)-thionophosphorsäureester(bekannt aus DE 21 11 414)

Beispiel A:

Phaedon-Larven-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration,

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 36, 52, 63.

Beispiel B:

Plutella-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 106.

**Patentansprüche**

1. Substituierte Pyrazoline der allgemeinen Formel (I)

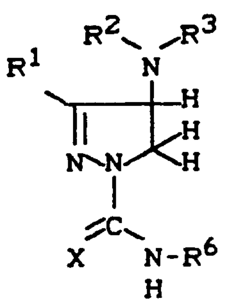

(I)

in welcher X, $R^1$, $R^2$, $R^3$ und $R^6$ jeweils folgende Bedeutungskombinationen einnehmen können:

| $R^1$ | $R^2$ | $R^3$ | $R^6$ | X |
|---|---|---|---|---|
| 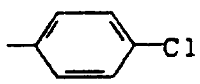 | 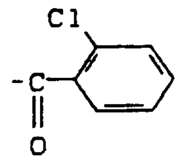 | H | 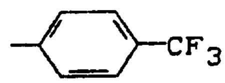 | O |
| 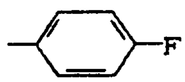 | 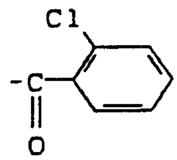 | H | 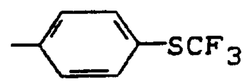 | O |
| 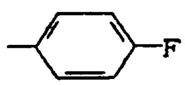 | 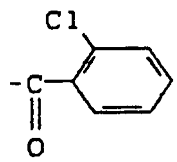 | H | 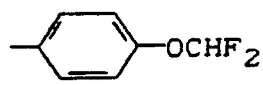 | O |
| 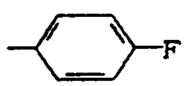 | 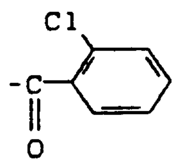 | H | 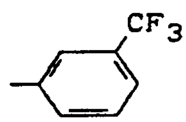 | O |
| 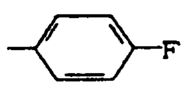 | 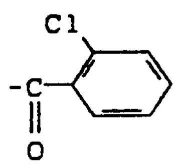 | H | 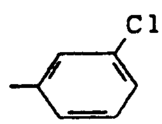 | O |

| $R^1$ | $R^2$ | $R^3$ | $R^6$ | X |
|---|---|---|---|---|
| 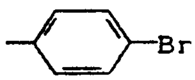 | 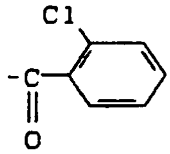 | H | 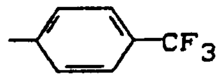 | O |
| 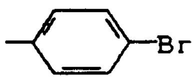 | 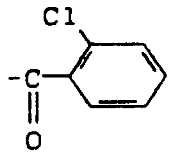 | H | 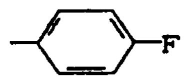 | O |
| 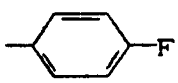 | 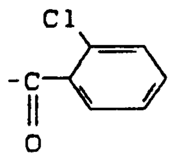 | H | 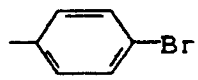 | O |
| 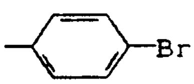 | 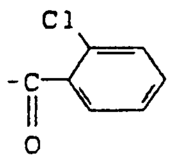 | H | 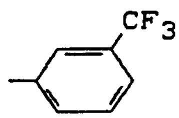 | O |
| 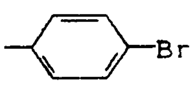 | 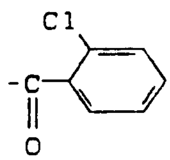 | H | 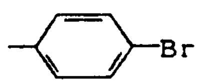 | O |
| 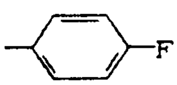 | 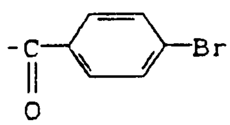 | H | 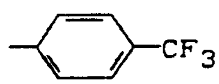 | O |
| 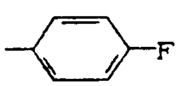 | 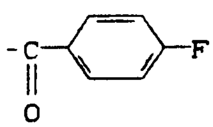 | H | 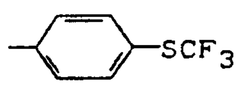 | O |
| 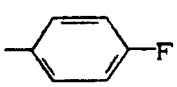 | 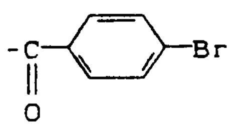 | H | 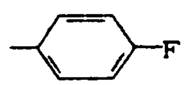 | O |
| 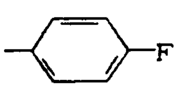 | 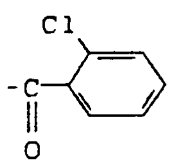 | H | 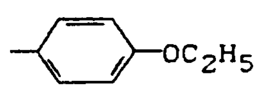 | O |

| $R^1$ | $R^2$ | $R^3$ | $R^6$ | X |
|---|---|---|---|---|
| 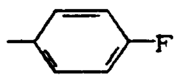 | 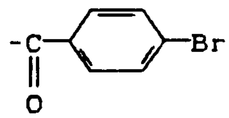 | H | 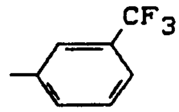 | O |
| 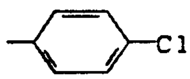 | 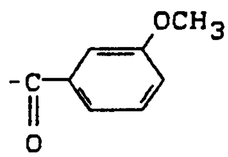 | H | 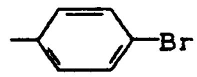 | O |
| 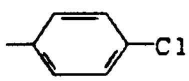 | 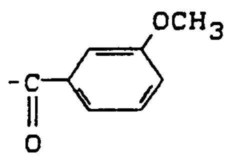 | H | 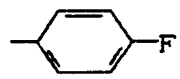 | O |
| 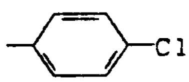 | 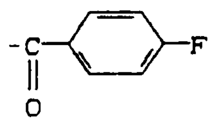 | H | 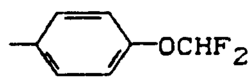 | O |
| 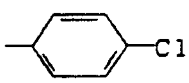 | 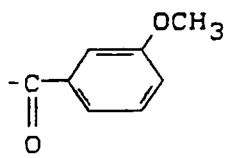 | H | 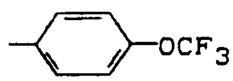 | O |
| 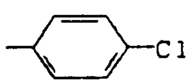 | 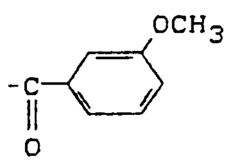 | H | 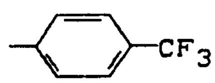 | O |
| 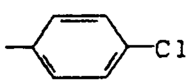 | 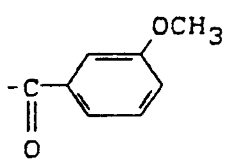 | H | 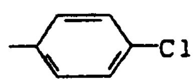 | O |
| 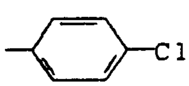 | 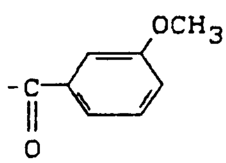 | H | 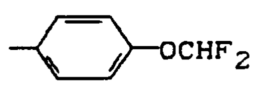 | O |
| 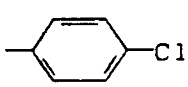 | 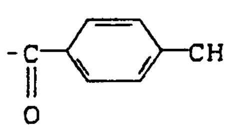 | H | 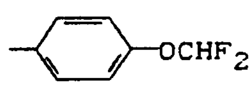 | O |

| $R^1$ | $R^2$ | $R^3$ | $R^6$ | X |
|---|---|---|---|---|
| 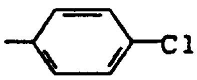 | 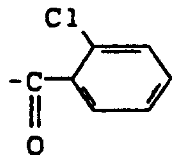 | H | 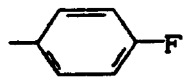 | O |
| 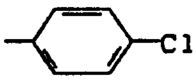 | 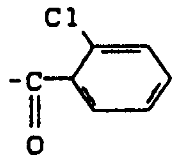 | H | 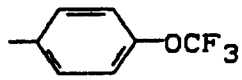 | O |
| 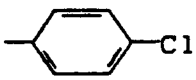 | 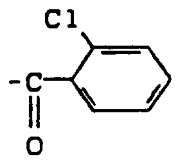 | H | 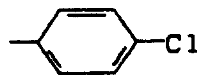 | O |
| 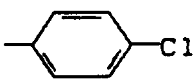 | 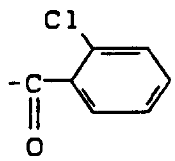 | H | 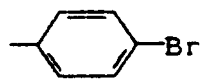 | O |
| 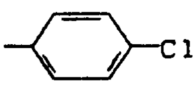 | 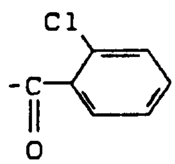 | H | 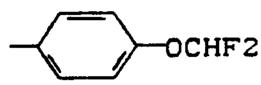 | O |
| 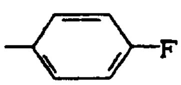 | 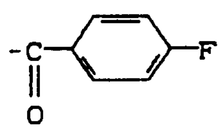 | H | 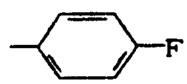 | O |
| 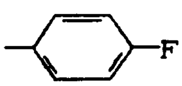 | 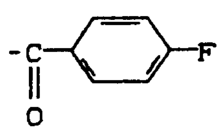 | H | 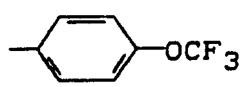 | O |
| 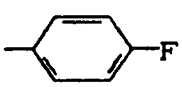 | 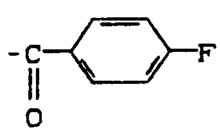 | H | 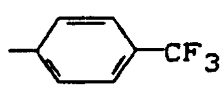 | O |

| $R^1$ | $R^2$ | $R^3$ | $R^6$ | X |
|---|---|---|---|---|
| 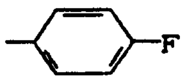 | 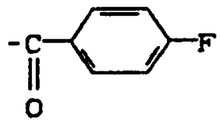 | H | 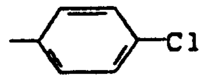 | O |
| 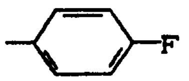 | 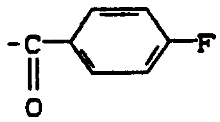 | H | 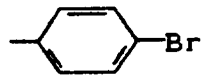 | O |
| 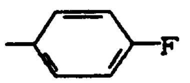 | 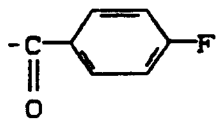 | H | 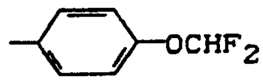 | O |
| 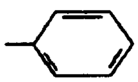 | 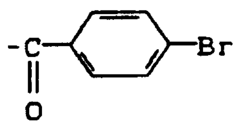 | H | 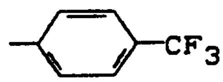 | O |
| 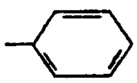 | 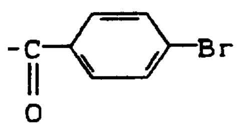 | H | 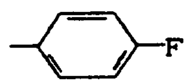 | O |
| 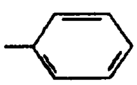 | 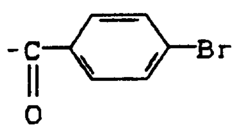 | H | 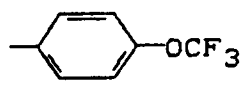 | O |
| 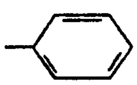 | 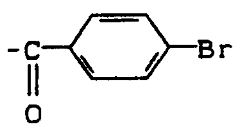 | H | 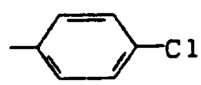 | O |
| 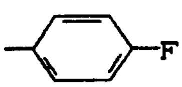 | 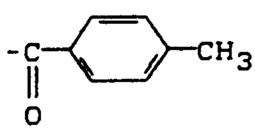 | H | 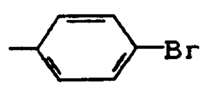 | O |
| 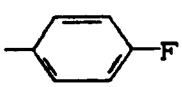 | 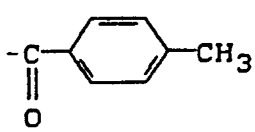 | H | 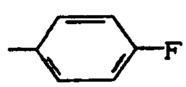 | O |

| $R^1$ | $R^2$ | $R^3$ | $R^6$ | X |
|---|---|---|---|---|
| 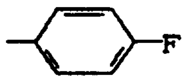 | 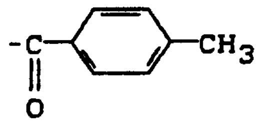 | H | 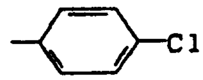 | O |
| 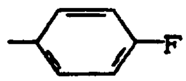 | 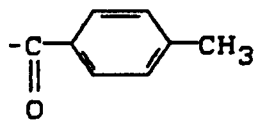 | H | 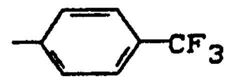 | O |
| 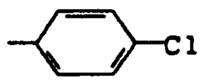 | 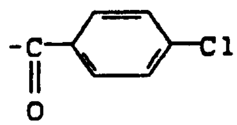 | H | 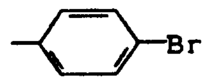 | O |
| 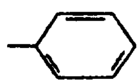 | 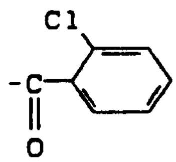 | H | 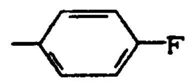 | O |
| 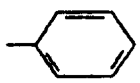 | 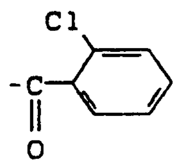 | H | 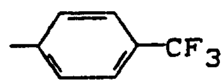 | O |
| 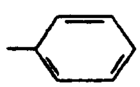 | 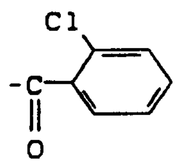 | H | 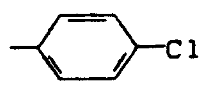 | O |
| 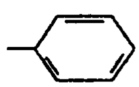 | 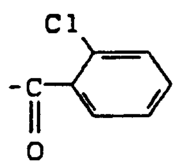 | H | 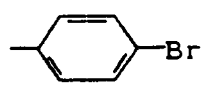 | O |
| 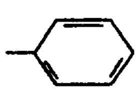 | 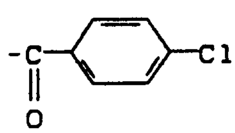 | H | 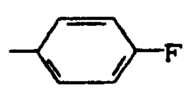 | O |

| $R^1$ | $R^2$ | $R^3$ | $R^6$ | X |
|---|---|---|---|---|
| 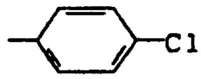 | 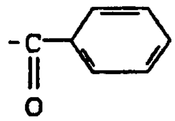 | H | 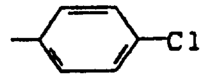 | O |
| 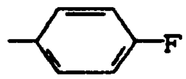 | 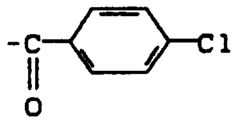 | H | 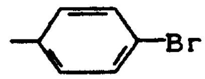 | O |
| 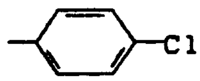 | 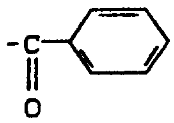 | H | 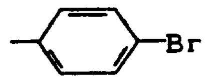 | O |
| 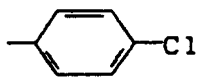 | 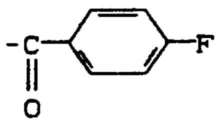 | H | 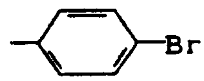 | O |
| 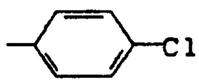 | 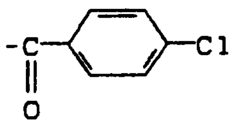 | H | 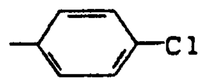 | O |
| 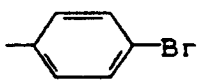 | 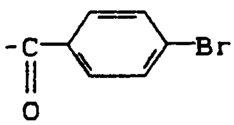 | H | 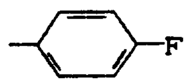 | O |
| 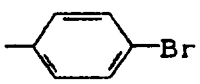 | 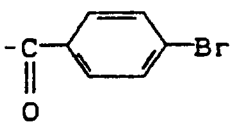 | H | 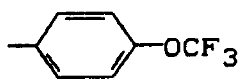 | O |
| 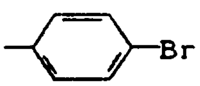 | 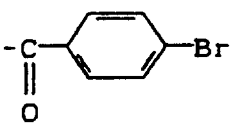 | H | 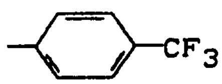 | O |
| 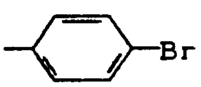 | 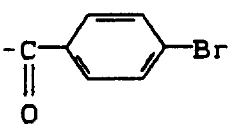 | H | 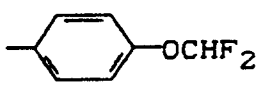 | O |

| $R^1$ | $R^2$ | $R^3$ | $R^6$ | X |
|---|---|---|---|---|
| 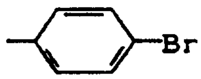 | 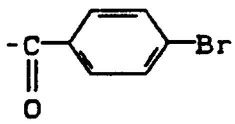 | H | 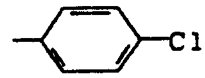 | O |
| 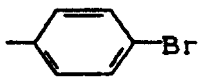 | 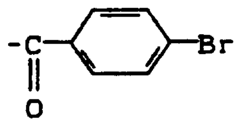 | H | 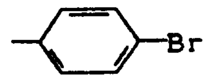 | O |
| 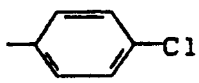 | 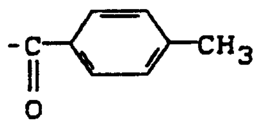 | H | 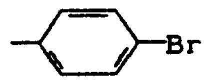 | O |
| 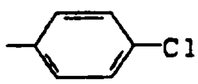 | 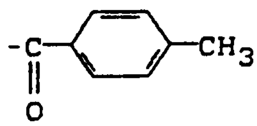 | H | 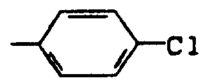 | O |
| 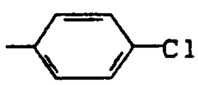 | 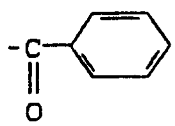 | H | 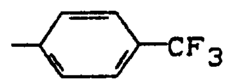 | O |
| 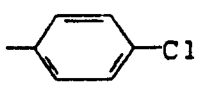 | 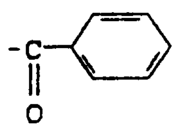 | H | 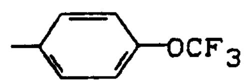 | O |
| 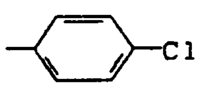 | 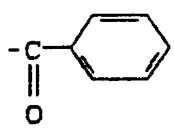 | H | 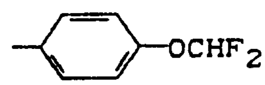 | O |
| 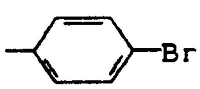 | 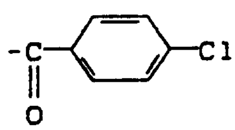 | H | 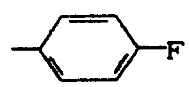 | O |
| 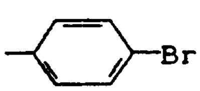 | 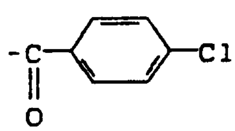 | H | 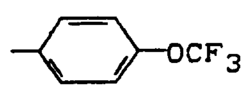 | O |

| $R^1$ | $R^2$ | $R^3$ | $R^6$ | X |
|---|---|---|---|---|
| 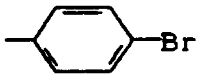 | 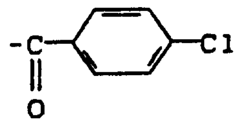 | H | 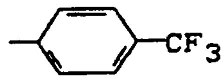 | O |
| 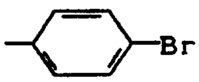 | 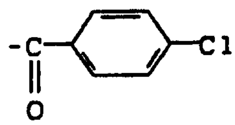 | H | 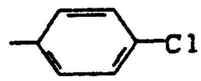 | O |
| 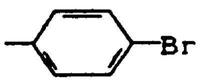 | 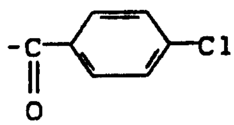 | H | 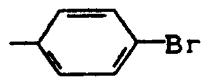 | O |
| 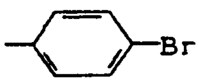 | 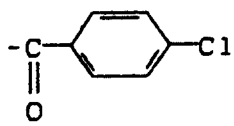 | H | 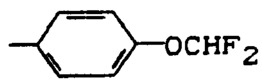 | O |
| 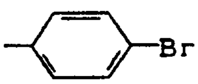 | 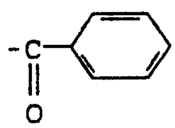 | H | 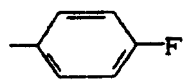 | O |
| 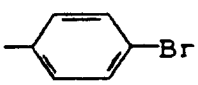 | 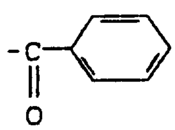 | H | 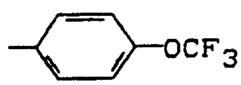 | O |
| 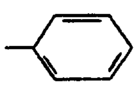 | 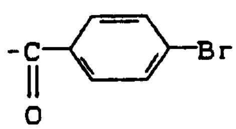 | H | 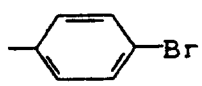 | O |
| 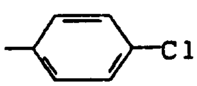 | 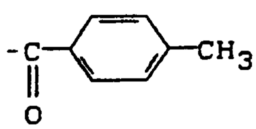 | H | 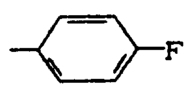 | O |
| 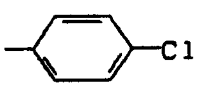 | 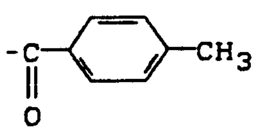 | H | 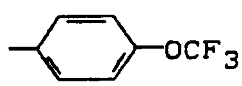 | O |

| $R^1$ | $R^2$ | $R^3$ | $R^6$ | X |
|---|---|---|---|---|
| 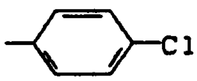 | 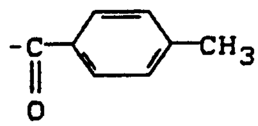 | H | 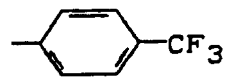 | O |
| 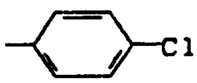 | 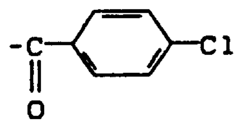 | H | 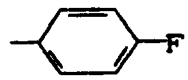 | O |
| 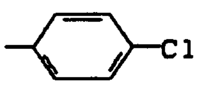 | 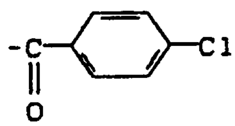 | H | 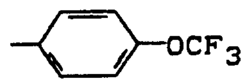 | O |
| 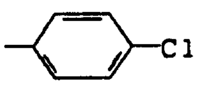 | 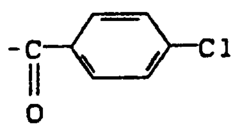 | H | 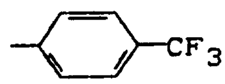 | O |
| 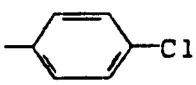 | 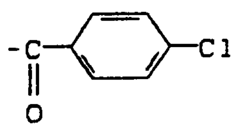 | H | 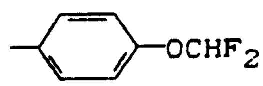 | O |
| 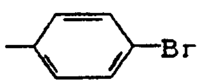 | 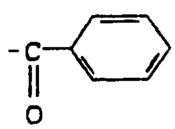 | H | 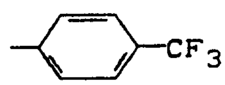 | O |
| 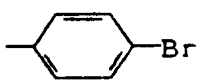 | 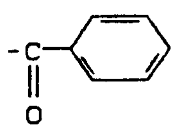 | H | 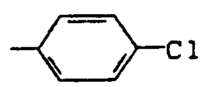 | O |
| 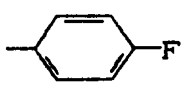 | 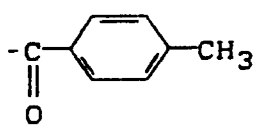 | H | 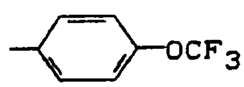 | O |
| 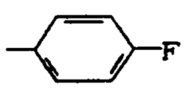 | 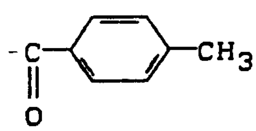 | H | 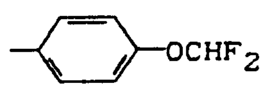 | O |

| $R^1$ | $R^2$ | $R^3$ | $R^6$ | X |
|---|---|---|---|---|
| 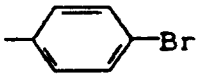 | 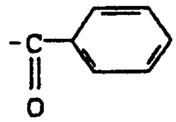 | H | 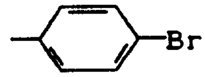 | O |
| 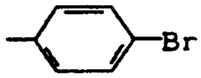 | 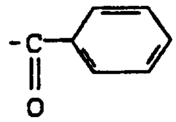 | H | 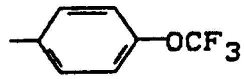 | O |
| 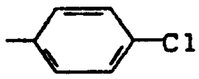 | 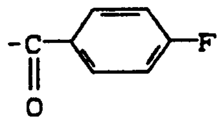 | H | 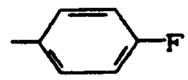 | O |
| 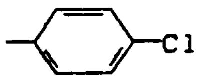 | 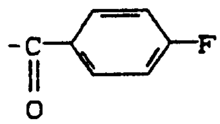 | H | 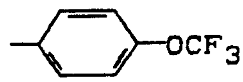 | O |
| 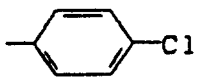 | 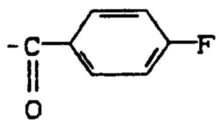 | H | 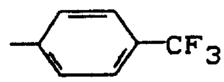 | O |
| 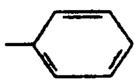 | 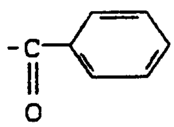 | H | 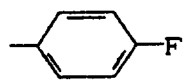 | O |
| 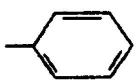 | 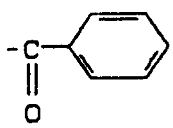 | H | 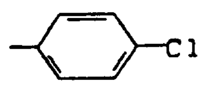 | O |
| 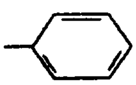 | 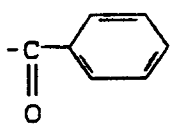 | H | 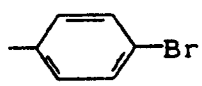 | O |
| 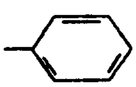 | 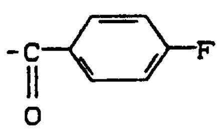 | H | 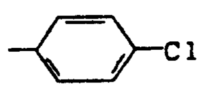 | O |

| $R^1$ | $R^2$ | $R^3$ | $R^6$ | X |
|---|---|---|---|---|
| 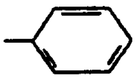 | 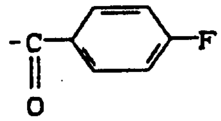 | H | 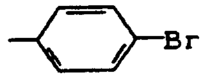 | O |
| 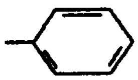 | 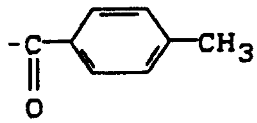 | H | 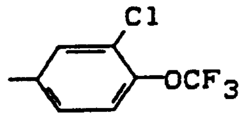 | O |
| 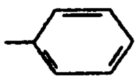 | 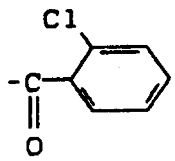 | H | 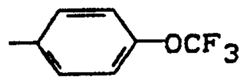 | O |
| 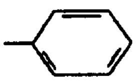 | 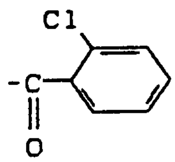 | H | 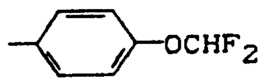 | O |
| 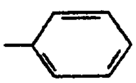 | 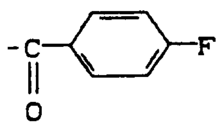 | H | 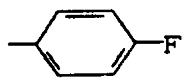 | O |
| 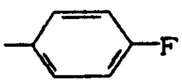 | 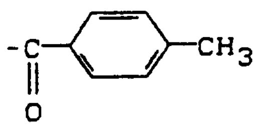 | H | 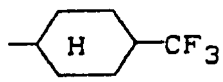 | O |
| 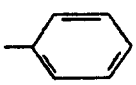 | 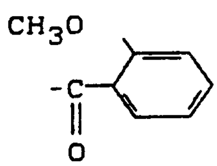 | H | 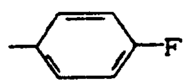 | O |
| 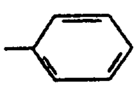 | 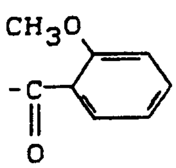 | H | 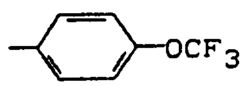 | O |

| $R^1$ | $R^2$ | $R^3$ | $R^6$ | X |
|---|---|---|---|---|
| 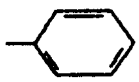 | 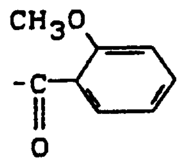 | H | 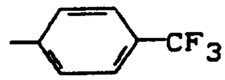 | O |
| 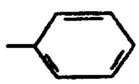 | 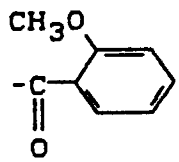 | H | 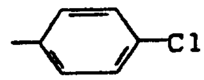 | O |
| 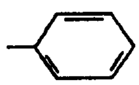 | 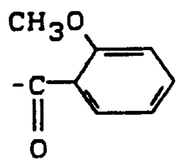 | H | 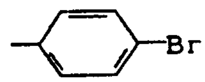 | O |
| 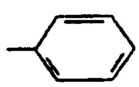 | 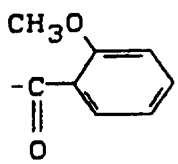 | H | 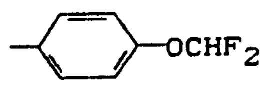 | O |
| 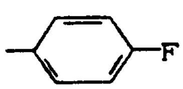 | 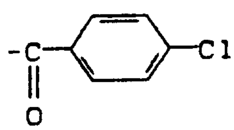 | H | 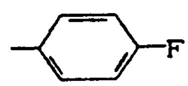 | O |
| 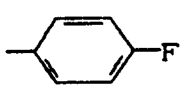 | 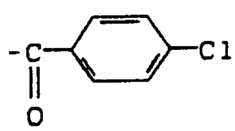 | H | 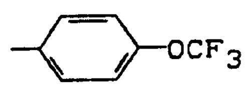 | O |
| 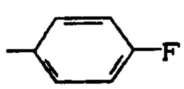 | 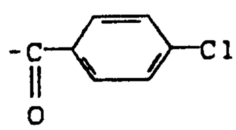 | H | 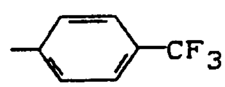 | O |
| 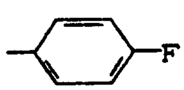 | 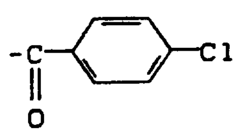 | H | 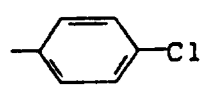 | O |

| $R^1$ | $R^2$ | $R^3$ | $R^6$ | X |
|---|---|---|---|---|
| 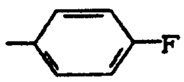 | 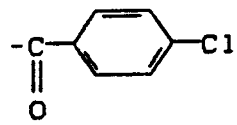 | H | 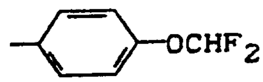 | O |
| 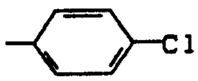 | 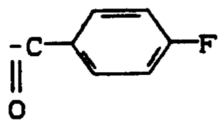 | H | 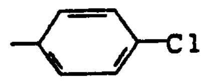 | O |
| 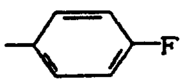 | 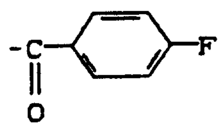 | H | 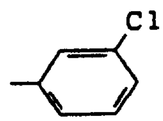 | O |
| 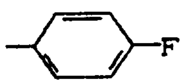 | 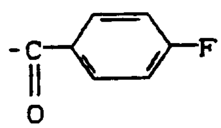 | H | 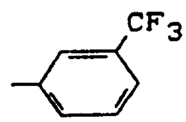 | O |
| 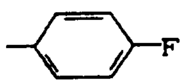 | 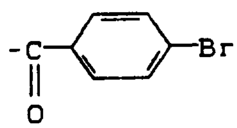 | H | 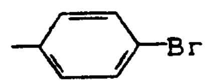 | O |
| 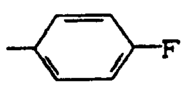 | 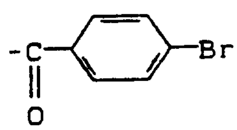 | H | 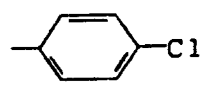 | O |
| 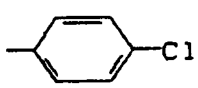 | 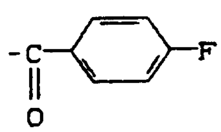 | H | 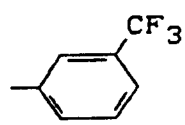 | O |
| 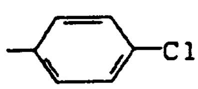 | 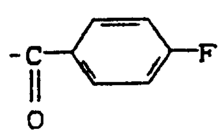 | H | 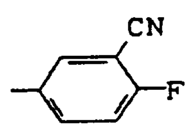 | O |

| $R^1$ | $R^2$ | $R^3$ | $R^6$ | X |
|---|---|---|---|---|
| 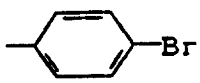 | 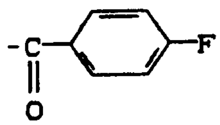 | H | 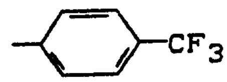 | O |
| 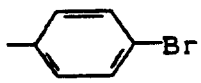 | 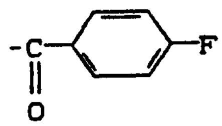 | H | 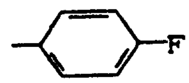 | O |
| 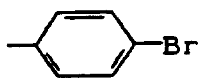 | 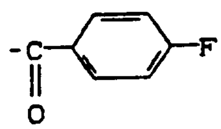 | H | 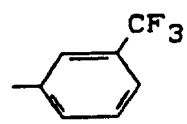 | O |
| 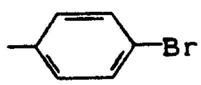 | 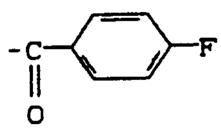 | H | 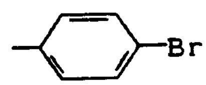 | O |
| 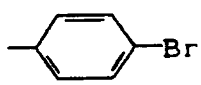 | 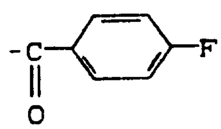 | H | 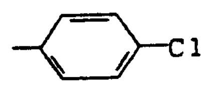 | O |
| 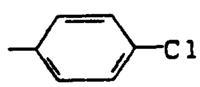 | 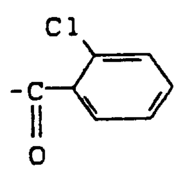 | H | 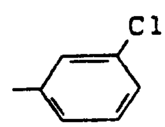 | O |
| 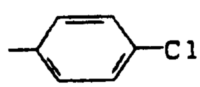 | 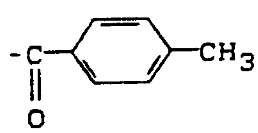 | H | 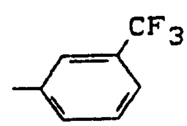 | O |
| 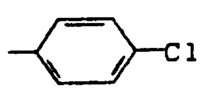 | 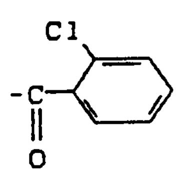 | H | 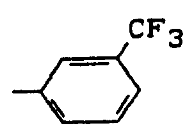 | O |

| $R^1$ | $R^2$ | $R^3$ | $R^6$ | X |
|---|---|---|---|---|
| 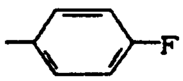 | 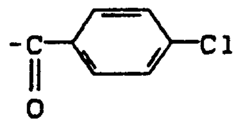 | H | 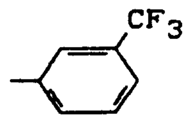 | O |
| 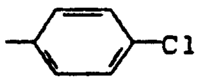 | 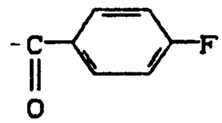 | H | 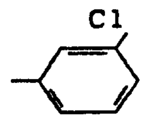 | O |
| 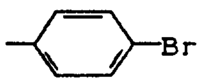 | | 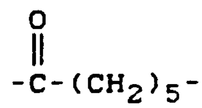 | 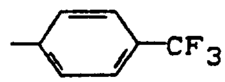 | O |
| 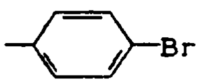 | 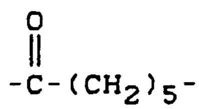 | | 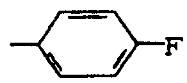 | O |
| 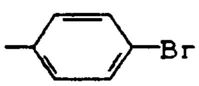 | 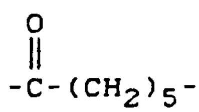 | | 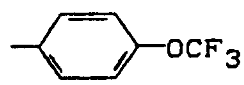 | O |
| 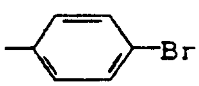 | 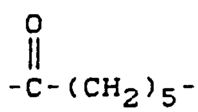 | | 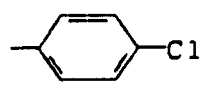 | O |
| 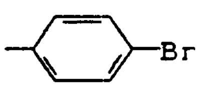 | 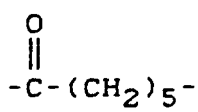 | | 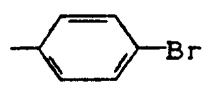 | O |
| 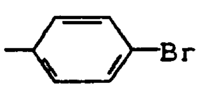 | 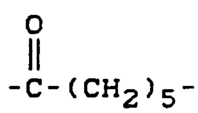 | | 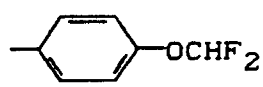 | O |

| $R^1$ | $R^2$ | $R^3$ | $R^6$ | X |
|---|---|---|---|---|
| 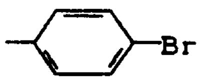 | 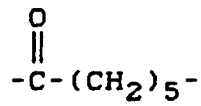 | | 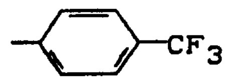 | O |
| 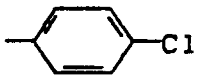 | 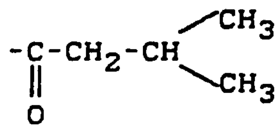 | H | 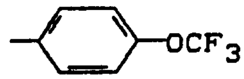 | O |
| 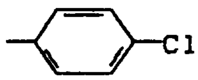 | 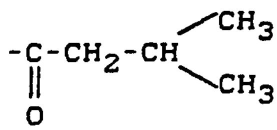 | H | 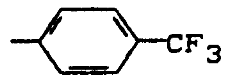 | O |
| 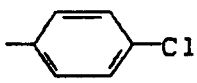 | 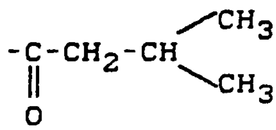 | H | 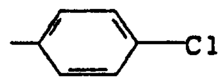 | O |
| 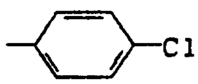 | 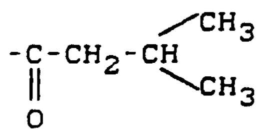 | H | 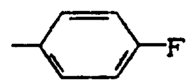 | O |
| 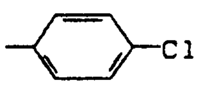 | 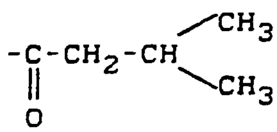 | H | 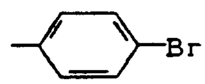 | O |
| 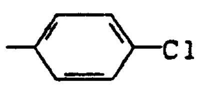 | 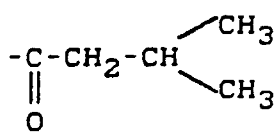 | H | 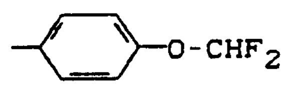 | O |
| 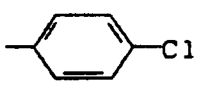 | 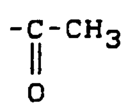 | H | 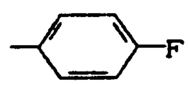 | O |
| 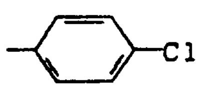 | 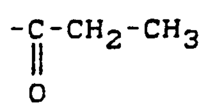 | H | 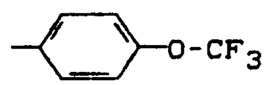 | O |

| $R^1$ | $R^2$ | $R^3$ | $R^6$ | X |
|---|---|---|---|---|
| 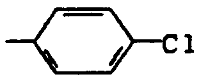 | 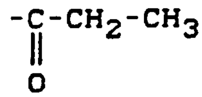 | H | 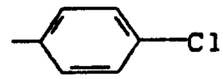 | O |
| 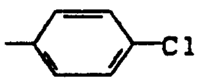 | 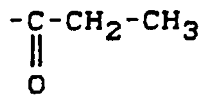 | H | 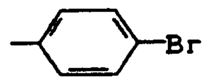 | O |
| 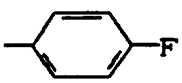 | 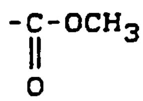 | H | 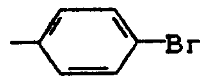 | O |
| 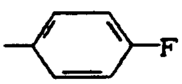 | 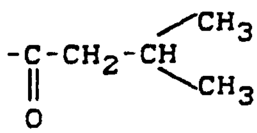 | H | 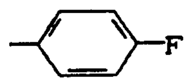 | O |
| 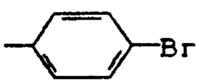 | | 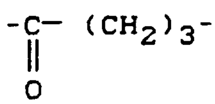 | 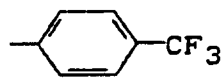 | O |
| 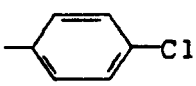 | 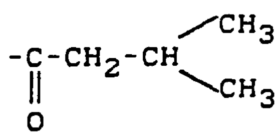 | H | 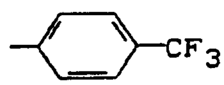 | O |
| 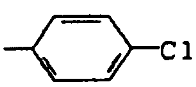 | 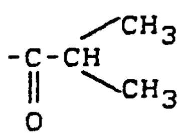 | H | 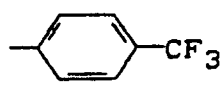 | O |
| 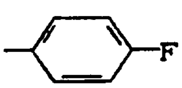 | 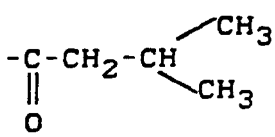 | H | 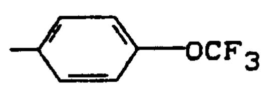 | O |
| 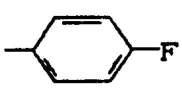 | 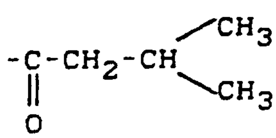 | H | 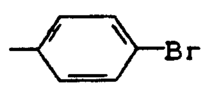 | O |

| $R^1$ | $R^2$ | $R^3$ | $R^6$ | X |
|---|---|---|---|---|
| 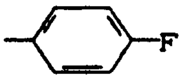 | 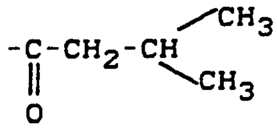 | H | 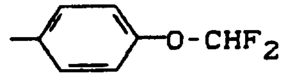 | O |
| 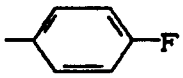 | 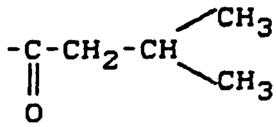 | H | 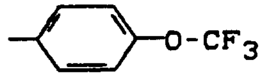 | O |
| 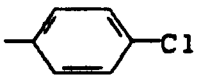 | 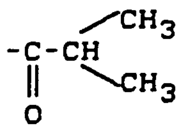 | H | 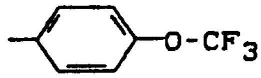 | O |
| 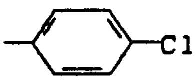 | 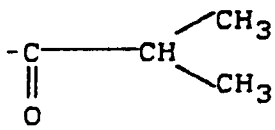 | H | 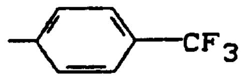 | O |
| 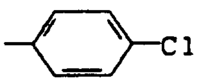 | 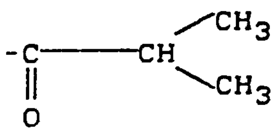 | H | 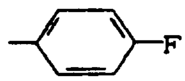 | O |
| 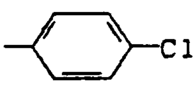 | 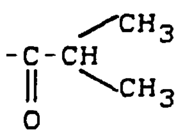 | H | 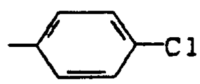 | O |
| 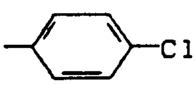 | 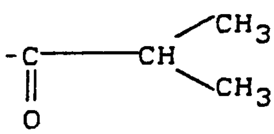 | H | 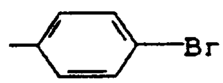 | O |
| 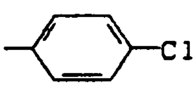 | 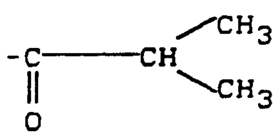 | H | 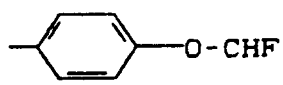 | O |
| 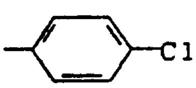 | 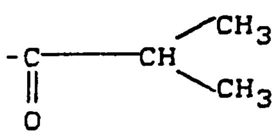 | H | 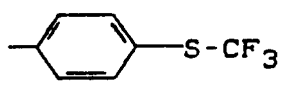 | O |

| $R^1$ | $R^2$ | $R^3$ | $R^6$ | X |
|---|---|---|---|---|
| 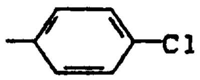 | 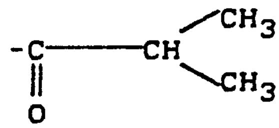 | H | 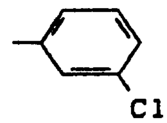 | O |
| 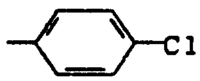 | 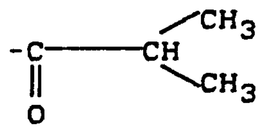 | H | 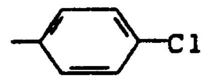 | O |
| 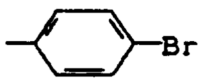 | | 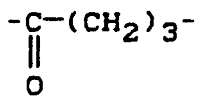 | 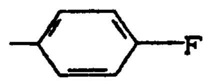 | O |
| 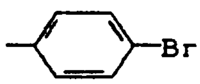 | | 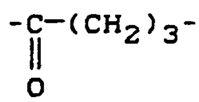 | 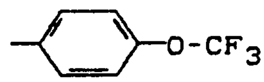 | O |
| 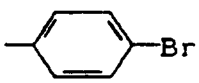 | | 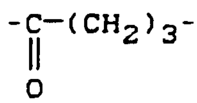 | 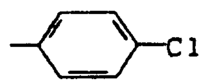 | O |
| 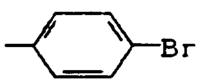 | 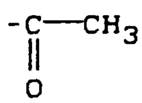 | H | 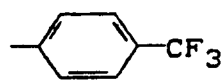 | O |
| 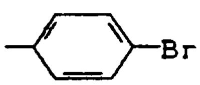 | 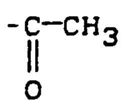 | H | 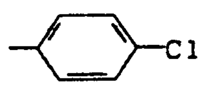 | O |
| 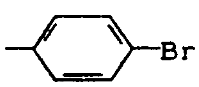 | 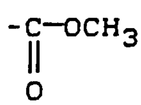 | H | 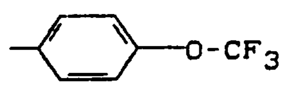 | O |
| 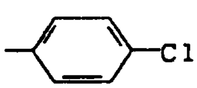 | 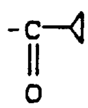 | H | 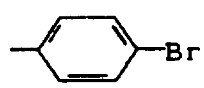 | O |

| $R^1$ | $R^2$ | $R^3$ | $R^6$ | X |
|---|---|---|---|---|
| 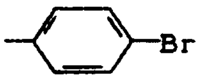 | | 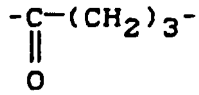 | 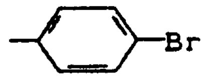 | O |
| 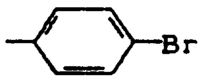 | | 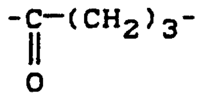 | 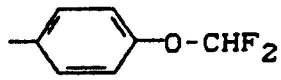 | O |
| 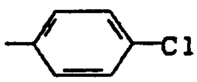 | 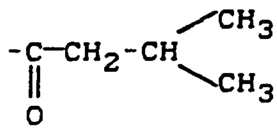 | H | 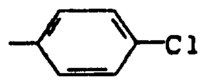 | O |
| 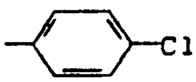 | 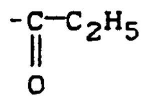 | H | 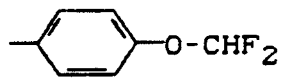 | O |
| 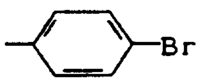 | 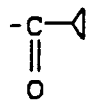 | H | 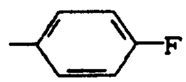 | O |
| 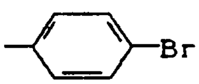 | 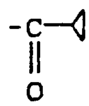 | H | 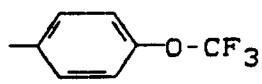 | O |
| 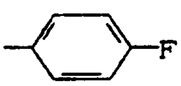 | 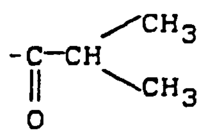 | H | 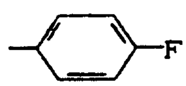 | O |
| 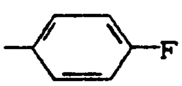 | 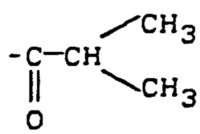 | H | 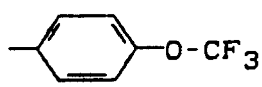 | O |
| 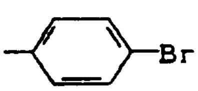 | 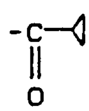 | H | 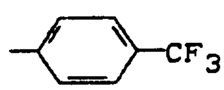 | O |

| $R^1$ | $R^2$ | $R^3$ | $R^6$ | X |
|---|---|---|---|---|
| 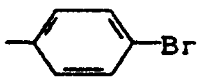 | 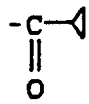 | H | 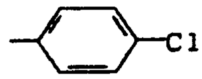 | O |
| 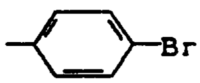 | 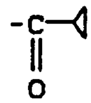 | H | 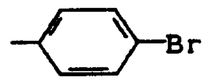 | O |
| 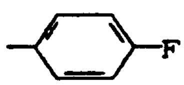 | 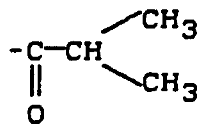 | H | 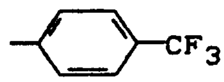 | O |
| 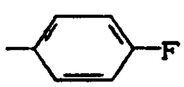 | 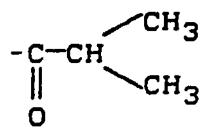 | H | 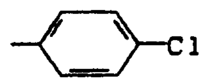 | O |
| 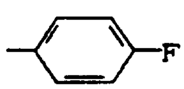 | 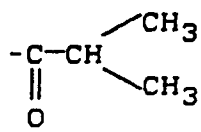 | H | 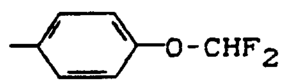 | O |
| 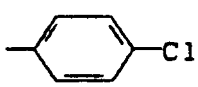 | 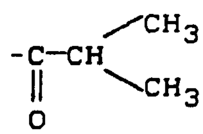 | H | 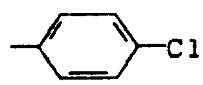 | S |
| 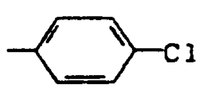 | 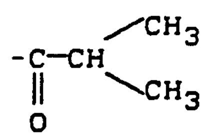 | H | 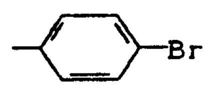 | S. |

2. Verfahren zur Herstellung von substituierten Pyrazolinen der allgemeinen Formel (I)

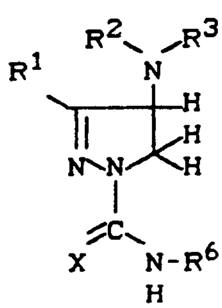

(I)

in welcher die Substituenten X, $R^1$, $R^2$, $R^3$ und $R^6$ die in Anspruch 1 aufgeführten Bedeutungskombinationen einnehmen können,

dadurch gekennzeichnet, daß man in 1-Stellung unsubstituierte Pyrazoline der Formel (II)

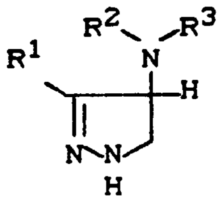

(II)

in welcher $R^1$, $R^2$, und $R^3$ die oben angegebene Bedeutung haben, entweder

a) mit Isocyanaten der Formel (III)

$R^6$-N=C=X (III)

in welcher $R^6$ die oben angegebene Bedeutung hat, oder

b) mit Carbaminsäurechloriden der Formel (IV)

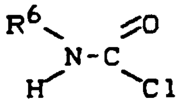

(IV)

in welcher $R^6$ die oben angegebene Bedeutung hat,

gegebenenfalls in Gegenwart eines Verdünnungsmittels und gegebenenfalls in Gegenwart eines Reaktionshilfsmittels umsetzt.

**3.** Schädlingsbekämpfungsmittel, gekennzeichnet durch einen Gehalt an mindestens einem substituierten Pyrazolin der Formel (I).

**4.** Verfahren zur Bekämpfung von tierischen Schädlingen, dadurch gekennzeichnet, daß man substituierte Pyrazoline der Formel (I) auf tierische Schädlinge und/oder ihren Lebensraum einwirken läßt.

**5.** Verwendung von substituierten Pyrazolinen der Formel (I) zur Bekämpfung von tierischen Schädlingen.

**6.** Verfahren zur Herstellung von Schädlingsbekämpfungsmitteln, dadurch gekennzeichnet, daß man substituierte Pyrazoline der Formel (I) mit Streckmitteln und/oder oberflächenaktiven Mitteln vermischt.

Europäisches Patentamt

# EUROPÄISCHER RECHERCHENBERICHT

Nummer der Anmeldung

EP 92 11 3957

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | EP-A-0 438 690 (BAYER AG)<br>--- | | C07D231/06<br>A01N47/38<br>C07D403/04 |
| D,A | US-A-4 174 393 (DUPHAR INTERNATIONAL RESEARCH B.V.)<br>--- | | |
| D,A | DE-A-2 700 258 (N.V. PHILIPS'GLOEILAMPENFABRIEKEN,)<br>--- | | |
| D,A | DE-A-2 529 689 (N.V. PHILIPS'GLOEILAMPENFABRIEKEN,)<br>--- | | |
| P,X | EP-A-0 466 408 (ROHM AND HAAS COMPANY)<br>----- | 1-6 | |
| | | | **RECHERCHIERTE SACHGEBIETE (Int. Cl.5 )** |
| | | | C07D<br>A01N |

Der vorliegende Recherchenbericht wurde für alle Patentansprüche erstellt

| Recherchenort | Abschlußdatum der Recherche | Prüfer |
|---|---|---|
| DEN HAAG | 09 DEZEMBER 1992 | DE BUYSER I.A.F. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)